# FASCICULE DE BREVET EUROPEEN

(11) **EP 1 036 090 B1**
(45) Date de publication et mention de la délivrance du brevet: **29.03.2006**
(21) Numéro de dépôt: 98959935.2
(22) Date de dépôt: 09.12.1998
(51) Int. Cl.: C07K 7/56, A61K 38/12

(54) **DERIVES DE L'ECHINOCANDINE, LEUR PROCEDE DE PREPARATION ET LEUR APPLICATION COMME ANTI-FONGIQUES**
ECHINOCANDINDERIVATE, DEREN HERSTELLUNG UND DEREN VERWENDUNG ZUR BEKÄMPFUNG VON PILZEN
ECHINOCANDIN DERIVATIVES, PREPARATION METHOD AND APPLICATION AS ANTI-FUNGAL AGENTS

(30) Priorité: 10.12.1997 FR 9715628; 26.10.1998 FR 9813361
(43) Date de publication de la demande: 20.09.2000
(73) Titulaire: NOVEXEL, 93230 Romainville (FR)
(72) Inventeur: COURTIN, Olivier, F-75012 Paris (FR); FAUVEAU, Patrick, F-93190 Livry Gargan (FR); MARKUS, Astrid, D-65835 Liederbach (DE); MELON MANGUER, Dominique, F-93100 Montreuil (FR); MICHEL, Jean-Marc, F-60200 Compiègne (FR); SCHIO, Laurent, F-93140 Bondy (FR)
(74) Mandataire: Hirsch & Associés
(86) Numéro de dépôt international: PCT/FR1998/002671
(87) Numéro de publication internationale: WO 1999/029716

(56) Documents cités:
- EP-A- 0 561 639
- EP-A- 0 736 541
- WO-A-96/08267
- WO-A-96/13272
- WO-A-96/22784
- GB-A- 2 241 955

## Description

La présente invention concerne de nouveaux dérivés des échinocandines, leur procédé de préparation et leur application comme antifongiques.

Les demandes de brevets EP-A-0736541, EP-A-0561639, GB-A-2241955, WO 9608267, WO 9613272 et WO 9622784 décrivent des dérivés de cyclohexapeptides présentant une activité antifongique.

L'invention a pour objet, sous toutes les formes d'isomères possibles ainsi que leurs mélanges, les composés de formule (I) : 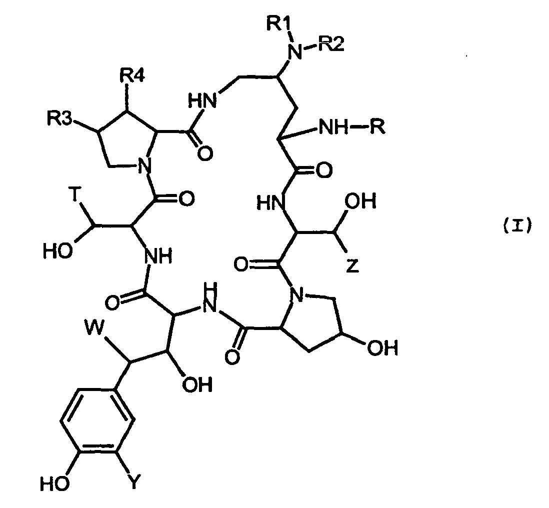 dans lesquels
ou bien R1 et R2 identiques ou différents l'un de l'autre, représentent un atome d'hydrogène, un radical hydroxyle, un radical alkyle renfermant jusqu'à 8 atomes de carbone liné-aire, ramifié ou cyclique, éventuellement interrompu par un atome d'oxygène éventuellement substitué par un atome d'halogène, un radical OH, un radical 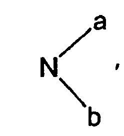 a et b identiques ou différents l'un de l'autre,
représentant un atome d'hydrogène ou un radical alkyle renfermant jusqu'à 8 atomes de carbone, a et b pouvant éventuellement former avec l'atome d'azote un hétérocycle renfermant éventuellement un ou plusieurs hétéroatomes supplémentaires,
- ou bien R1 forme avec l'atome de carbone endocyclique portant le radical 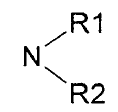 une double liaison et ou bien R2 représente un radical XRₐ, X représentant un atome d'oxygène
ou un radical NH ou N-alkyle renfermant jusqu'à 8 atomes de carbone et Rₐ représente un atome d'hydrogène, un radical alkyle linéaire, ramifié ou cyclique renfermant jusqu'à 8 atomes de carbone éventuellement substitué par un ou plusieurs atomes d'halogène, par un ou plusieurs radicaux OH, CO₂H, CO₂alc,
par un radical 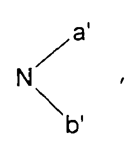 a' et b' représentant un atome d'hydrogène, un radical alkyle renfermant jusqu'à 8 atomes de carbone, a' et b' pouvant former un hétérocycle renfermant éventuellement un ou plusieurs hétéroatomes supplémentaires et/ou par un hétérocycle renfermant un ou plusieurs hétéro-atomes ou R2 représente un radical 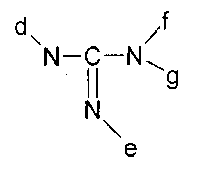 dans lequel d, e, f et g représentent un atome d'hydrogène ou un radical alkyle renfermant jusqu'à 8 atomes de carbone, f et g pouvant en outre représenter un radical acyle renfermant jusqu'à 8 atomes de carbone, e et f pouvant également former un cycle renfermant éventuellement un ou plusieurs hétéro-atomes,
R3 représente un atome d'hydrogène, un radical méthyle ou hydroxyle
R4 représente un atome d'hydrogène ou un radical hydroxyle
R représente une chaîne linéaire ou ramifiée ou cyclique renfermant jusqu'à 30 atomes de carbone, renfermant éventuellement, un ou plusieurs hétéroatomes, un ou plusieurs hétérocycles ou un radical acyle linaire, ramifié ou cyclique renfermant jusqu'à 30 atomes de carbone renfermant éventuellement un ou plusieurs hétéroatomes et/ou un ou plusieurs hétérocycles,
T représente un atome d'hydrogène, un radical méthyle, un radical CH₂CONH₂, CH₂C≡N, un radical (CH₂) ₂NH₂ ou (CH₂)₂Nalc₂⁺X⁻, X étant un atome d'halogène et alc un radical alkyle renfermant jusqu'à 8 atomes de carbone,
Y représente un atome d'hydrogène, un radical hydroxyle ou un atome d'halogène ou un radical OSO3H ou l'un des sels de ce radical,
W représente un atome d'hydrogène ou un radical OH,
Z représente un atome d'hydrogène ou un radical méthyle,
ainsi que les sels d'addition avec les acides des produits de formule (I) .

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, trifluoroacétique, propionique, benzoique, maléique, fumarique, succinique, tartrique, citrique, oxalique, glyoxy-lique, aspartique, alcanesulfoniques, tels que les acides méthane ou éthane sulfoniques, arylsulfoniques tels que les acides benzène ou paratoluènesulfoniques.

Dans la définition des substituants,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclo-hexyle,
- l'halogène est de préférence le fluor ou le chlore ou le brome,
- le radical aryle est de préférence le radical phényle,
- le radical hétérocyclique est de préférence le radical pyrrolyle, pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle, aziridinyle.

Comme sel du radical SO₃H on peut citer les sels de sodium, de potassium ou encore les sels d'amines.

Parmi les composés préférés de l'invention, on peut citer tout particulièrement :
- les composés de formule (I), dans lesquels T représente un atome d'hydrogène,
- les composés de formule (I), dans lesquels Y représente un atome d'hydrogène,
- les composés de formule (I), dans lesquels W représente un atome d'hydrogène,
- les composés de formule (I), dans lesquels Z représente un radical méthyle,
- les composés de formule (I), dans lesquels R3 représente un radical méthyle,
- les composés de formule (I), dans lesquels R4 représente un radical hydroxyle
- les composés de formule (I), dans lesquels R représente un radical 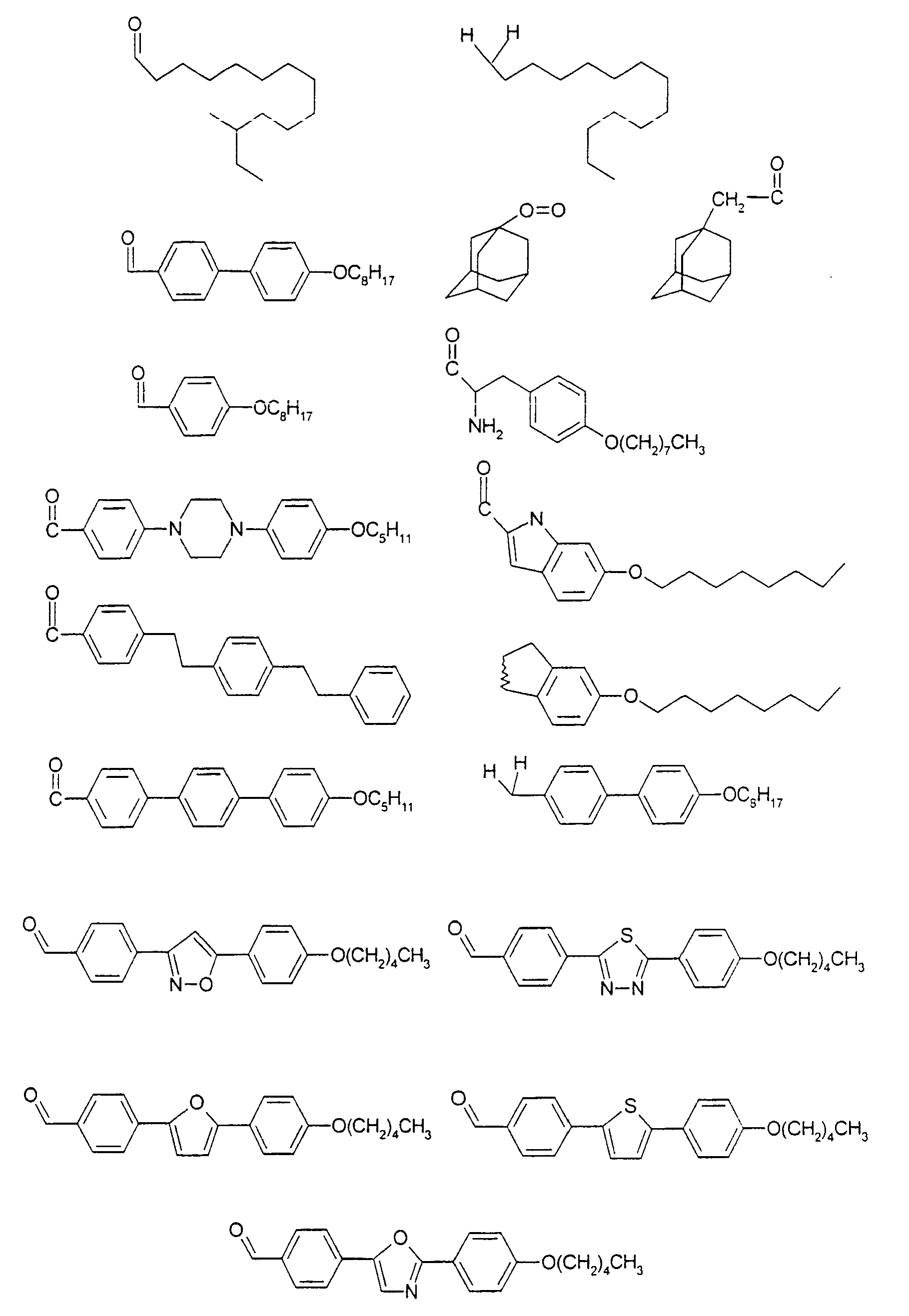 et tout particulièrement ceux dans lesquels R représente une chaîne 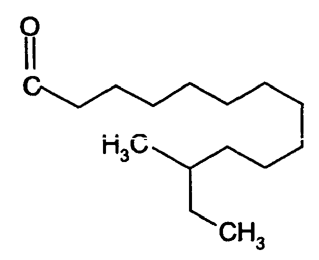 ou ceux dans lesquels R représente une chaîne 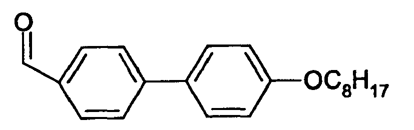
- les composés de formule (I) dans lesquels R1 forme avec l'atome de carbone endocyclique portant le radical NR1R2, une double liaison, et notamment ceux dans lesquels R2 représente le radical

   O(CH₂)ₙNY'₂

   dans lequel n représente un nombre entier compris entre 1 et 8 et tout spécialement ceux dans lesquels n représente le nombre 2 et Y' représente un atome d'hydrogène ou un radical alkyle renfermant jusqu'à 8 atomes de carbone, et ceux dans lesquels R2 représente un radical

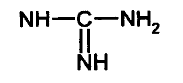

L'invention a également tout particulièrement pour objet les composés de formule (I) dans lesquels R2 représente un radical

(CH₂)ₚNY"₂

dans lequel Y" représente un atome d'hydrogène ou un radical alkyle renfermant jusqu'à 8 atomes de carbone et p représente un nombre entier variant de 1 à 8, et tout spécialement les composés dans lesquels p représente le nombre 2.

L'invention a tout spécialement pour objet, les composés dans lesquels R1 représente un atome d'hydrogène.

Parmi les composés préférés de l'invention, on peut citer les produits des exemples 8, 9, 11, 13 et 14.

Les composés de formule (I) présentent d'intéressantes propriétés antifongiques ; ils sont notamment actifs sur Candida albicans et autres Candida comme Candida glabrata, krusei, tropicalis, pseudotropicalis, parapsilosis et Aspergillus fumigatus, Aspergillus flavus, Cryptococcus neoformans.

Les composés de formule (I) peuvent être utilisés en tant que médicaments chez l'homme ou l'animal, pour lutter notamment contre les candidoses digestives, urinaires, vaginales ou cutanées, les cryptococcoses, par exemple les cryptococcoses neuroménengées, pulmonaires ou cutanées, les aspergilloses bronchopulmonaires et pulmonaires et les aspergilloses invasives de l'immunodéprimé.

Les composés de l'invention peuvent être utilisés également dans la prévention des affections mycosiques chez les déprimés immunitaires congénitaux ou acquis.

Les composés de l'invention ne sont pas limités à une utilisation pharmaceutique, ils peuvent être également utilisés comme fongicides dans d'autres domaines que pharmaceutiques.

L'invention a donc pour objet à titre de composés antifongiques, les composés de formule (I) ainsi que leurs sels d'addition avec les acides.

L'invention a également pour objet les composés de formule (I), à titre de médicaments:

L'invention a tout particulièrement pour objet les compositions pharmaceutiques renfermant comme principe actif au moins un composé de formule (I) ou l'un de ses sels d'addition avec les acides pharmaceutiquement acceptables.

Ces compositions peuvent être administrées par voie buccale, rectale, parentérale ou par voie locale en application topique sur la peau et les muqueuses, mais la voie préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceuti ques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour les produits des exemples 8, 9, 11, 13 et 14.

L'invention a également pour objet un procédé de préparation des composés de formule (I), caractérisé en ce que l'on soumet un composé de formule (II) : 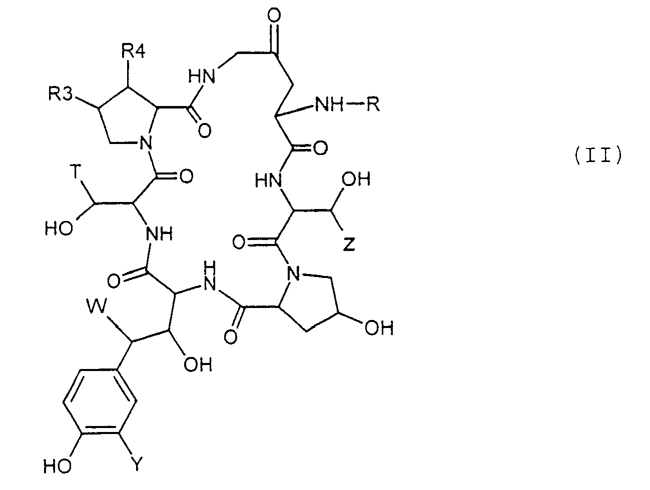 dans laquelle R, R3, R4, T, W, Y et Z conservent leur signification précédente, à l'action d'une amine ou d'un dérivé d'amine susceptible d'introduire
le radical 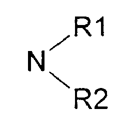 dans lequel R1 et R2 conservent leur signification précédente et si désiré à l'action d'un agent de réduction
et/ou d'un agent de fonctionnalisation de l'amine,
et/ou d'un acide pour former le sel du produit obtenu,
et/ou d'un agent de séparation des différents isomères obtenus,
et obtient ainsi le composé de formule (I) recherché 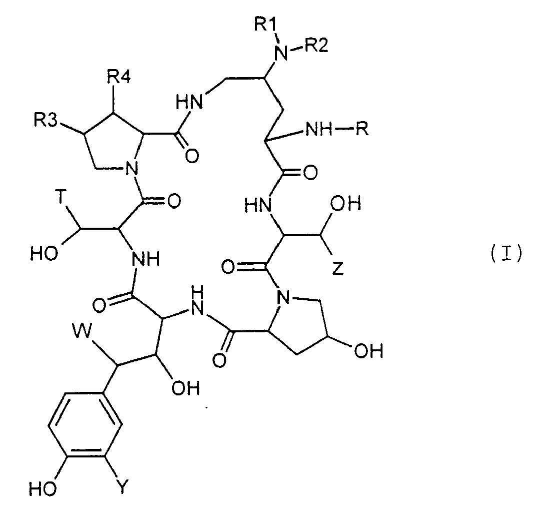 dans laquelle R1, R2, T, W, Y, R et Z conservent leur signification précédente sous toutes ses formes d'isomères possibles ainsi que leurs mélanges et/ou sous forme de sels avec les acides.

Les composés de formule (II) utilisés comme composés de départ du procédé de l'invention sont des produits nouveaux et sont eux-mêmes un objet de la présente invention, leur préparation donnée en partie expérimentale peut être schématisée comme suit : 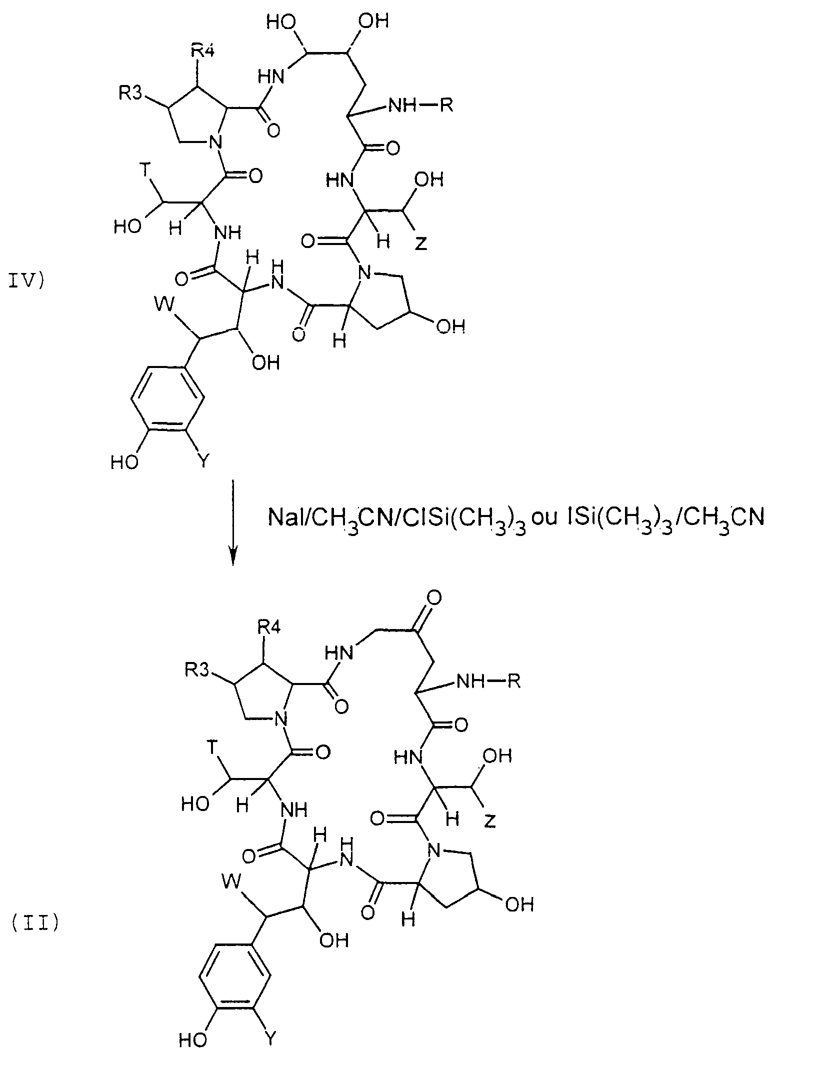 On peut utiliser ISi-(CH₃)₃ ou tout autre acide de Lewis.

Il est donné dans la partie expérimentale un exemple détaillé de préparation de composé de formule (II), et l'invention a plus particulièrement pour objet à titre de produit chimique nouveau le 1-[4-oxo-N2-(12-méthyl-1-oxotétradécyl) L-ornithine] 4-[4-(4-hydroxyphényl)-L-thréonine]-5-L-sérine-échinocandine B.

Le produit (IV) correspondant au produit de départ de la préparation 1 est un produit connu décrit et revendiqué dans le brevet européen 438813.

Les exemples suivants illustrent l'invention sans toutefois la limiter.

L'invention a également pour objet un procédé de préparation caractérisée en ce que l'on soumet un composé de formule (III) 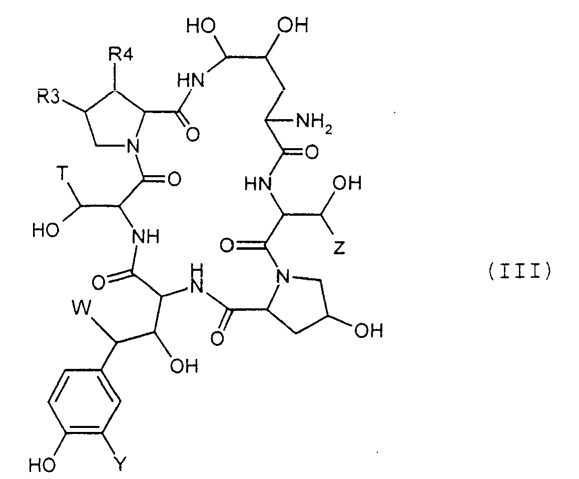 dans laquelle les différents substituants conservent leur signification précédente à l'action d'un agent capable de remplacer NH₂ par NHR, R conservant sa signification précédente pour obtenir le composé de formule (IV) 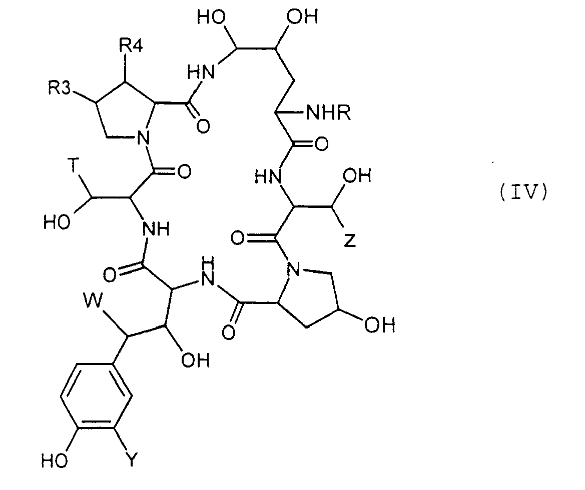 dans lesquels les différents substituants conservent leur signification précédente que l'on soumet à l'action de l'iodure de triméthyl silyle pour obtenir le composé de formule (II).

### PREPARATION 1 : 1-[N2-(12-méthyl-1-oxotétradécyl)-4-oxo-L-ornithine] 4- [4- (4-hydroxyphényl)-L-thréonine]-5-L-sérine échinocandine B.

On introduit sous agitation magnétique et sous atmosphère d'azote 1 g de 1-[(4R,5R)-4,5-dihydroxy-N2-(12-méthyl-1-oxotétradécyl)-L-ornithine] 4-[4-(4-hydroxyphényl)-L-thréonine]-5-L-sérine échinocandine B dans 25 ml d'acétonitrile. On ajoute 455 µl d'iodure de triméthylsilyle. On chauffe à 55°C pendant 40 minutes. On hydrolyse avec une solution de thiosulfate de sodium à 3%. Après 10 minutes sous agitation, on amène à sec sous pression réduite et purifie par chromatographie sur silice. On obtient 62% de produit recherché.
CCM : rf = 0,25 (éluant : CH₂Cl₂-MeOH-H₂O 86-13-1).

### EXEMPLE 1 : Trifluoroacétate de 1-[4-amino-N2-(12-méthyl-1-oxotétradécyl)-L-ornithine] 4-[4-(4-hydroxyphényl)-L-thréonine]-5-L-sérine échinocandine B (isomère B).

On introduit 50 mg du produit de la préparation 1 dans 2,5 ml de méthanol en présence de siliporite activé 4Å. On ajoute à 20°C, 158 mg d'acétate d'ammonium. On chauffe la solution obtenue à 50°C et ajoute 5,5 mg de NaBH₃CN. On agite pendant 3 heures 15 minutes. On ajoute 1 ml d'eau déminéralisée et concentre à sec la solution. On obtient 166 mg de produit que l'on purifie par HPLC (C₁₈) en éluant avec le mélange CH₃CN-H₂O-TFA (50-50-0,02). On obtient 17 mg de produit recherché.
MH⁺ = 975.

### EXEMPLE 2 : Trifluoroacétate de 1-[4-[[2-diméthylaminoéthylamino-N2-(12-méthyl-1-oxotétradécyl)-L-ornithine] 4-[4-(4-hydroxyphényl)-L-thréonine]-5-L-sérine échinocandine B (isomères A et B).

On introduit, à 20°C, 80 mg du produit de la préparation 1 dans une solution renfermant 1 ml de méthanol, 160 µl de 2-diméthyl-aminoéthylamine, 8 ml d'une solution 1M d'acide chlorhydrique dans le méthanol en présence de siliporite 4 Å. On introduit 35 mg de cyanoborohydrure de sodium et agite 20 heures à 20°C. On filtre, lave au méthanol et concentre à sec. On obtient 325 mg de produit que l'on purifie par HPLC (C₁₈) (éluant : CH₃CN-H₂O-TFA 45-55-0, 02 puis CH₃CN-H₂O-TFA 42-58-0,02). On obtient 8,1 mg de produit recherché isomère A et 9,4 mg de produit recherché isomère B.
Spectre de Masse :
MH⁺ = 1046
MNa⁺ = 1068.

### EXEMPLE 3 : Trifluoroacétate de 1-[4-[(3-aminopropyl)amino]-N2-(12-méthyl-1-oxotétradécyl)-L-ornithine] 4-[4-(4-hydroxyphényl)-L-thréonine]-5-L-sérine échinocandine B (isomères A et B).

On ajoute à 0°C 30 cm³ d'une solution 1M d'acide chlorhydrique dans le méthanol dans une solution renfermant 200 mg de produit de la préparation 1, 2 ml de méthanol et 300 µl de diaminopropane. On agite pendant 15 minutes à 0°C et ajoute 84 mg de cyanoborohydrure de sodium à 95%. On laisse 6 heures sous agitation à la température ambiante et amène à sec sous pression réduite. On empâte le résidu obtenu dans l'acétonitrile, essore et sèche sous pression réduite. On obtient 312 mg de produit que l'on purifie par HPLC (C₁₈) (éluant : CH₃CN-H₂O-TFA 45-55-0,02) et obtient 15 mg d'isomère A et 10 mg d'isomère B.
Spectre de masse :
MH⁺ = 1032.

### EXEMPLE 4 : (Z + E) Trifluoroacétate de 1-[4-[(4,5-dihydro-1H-imidazol-2-yl)hydrazono]-N2-(12-méthyl-1-oxotétradécyl)-L-ornithine] 4-[4-(4-hydroxyphényl)-L-thréonine]-5-L-sérine échinocandine B.

On maintient pendant 2 heures, sous agitation, au reflux 350 mg de produit de la préparation 1, 12 ml de méthanol et 130 mg de bromhydrate de 2-hydrazino 2-imidazoline. Après évaporation à sec, on obtient 510 mg de produit que l'on purifie par chromatographie sur silice en éluant avec le mélange CH₂Cl₂-MeOH-H₂O (86-13-1) puis par HPLC semi-préparative (C₁₈) en éluant avec le mélange CH₃CN-H₂O-TFA (55-45-0,02). On obtient ainsi 133 mg de produit recherché.
Spectre de masse :
MH⁺ = 1056
MNa⁺ = 1078.

### EXEMPLE 5 : (Z) 1-[4-[(2-hydroxyéthoxy) imino]-N2-(12-méthyl-1-oxotétradécyl)-L-ornithine] 4-[4-(4-hydroxyphényl)-L-thréonine]-5-L-sérine échinocandine B et isomère E correspondant.

On maintient pendant 4 heures au reflux un mélange de 36 mg de O-(2-hydroxyéthyl) hydroxylamine, 5 ml d'éthanol, 12 µl de pyridine, 4 µl d'acide acétique pur et 150 mg du produit de la préparation 1. On obtient 205 mg de produit que l'on purifie par chromatographie sur silice en éluant avec le mélange chlorure de méthylène-méthanol-eau (86-13-1). On isole 2 produits de rf = 0,2 et 0,25 (isomère Z et isomère E).
Spectre de masse :
MH⁺ = 1033
MNa⁺ = 1055.

### EXEMPLE 6 : (E) 1-[4-(hydroxyimino)-N2-(12-méthyl-1-oxotétradécyl)-L-ornithine] 4-[4-(4-hydroxyphényl)-L-thréonine]-5-L-sérine échinocandine B et isomère Z correspondant.

On laisse 1 heure sous agitation au reflux un mélange renfermant 200 mg du produit de la préparation 1, 8 ml d'éthanol, 36 mg de chlorhydrate d'hydroxylamine. On amène à sec et purifie par chromatographie HPLC (C₁₈) (éluant CH₃CN-H₂O 60-40). On obtient 72 mg d'isomère Z et 60 mg d'isomère E.
Spectre de masse :
MH⁺ = 989
MNa⁺ = 1011

### EXEMPLE 7 : Trifluoroacétate de 1-[4-(hydroxyamino)-N2-(12-méthyl-1-oxotétradécyl)-L-ornithine] 4-[4-(4-hydroxyphényl)-L-thréonine]-5-L-sérine échinocandine B (isomère A et isomère B).

On laisse 3 heures sous agitation 70 mg de mélange d'oximes E + Z obtenu à l'exemple précédent, 1 cm³ d'acide trifluoroacétique, 12 mg de cyanoborohydrure de sodium à 95%. On amène à sec sous pression réduite. On purifie par HPLC (C₁₈). On obtient les produits recherchés.
Spectre de masse :
MH⁺ = 991
MNa⁺ = 1013

### EXEMPLE 8 : (Z) Chlorhydrate de 1-[(S)-N2-(12-méthyl-1-oxotétradécyl) 4-[[(3-pipéridinyl) oxy] imino]-L-ornithine] 4-[4-(4-hydroxyphényl)-L-thréonine]-5-L-sérine échinocandine B.

### Stade A :

On ajoute 146 mg de produit de la préparation 1 et 60 µl d'acide acétique dans une solution renfermant 45 mg de R-3-(aminooxy)-1-pipéridine carboxylate de phénylméthyle et 2 ml de méthanol. On agite pendant 2 heures à la température ambiante. On concentre, purifie par chromatographie sur silice en éluant avec le mélange chlorure de méthylène-méthanol 98-2. On obtient ainsi le produit recherché.
Spectre de masse :
MH⁺ = 1206
MNa⁻ = 1228

### Stade B :

On met sous atmosphère d'hydrogène et vive agitation pendant 5 heures un mélange renfermant 61 mg de produit préparé au stade A, 20 mg de palladium sur charbon et 1 ml d'acide acétique. On filtre et concentre. On obtient 65% de produit recherché.
Spectre de masse :
MH⁺ = 1072.

### EXEMPLE 9 : Trifluoroacétate de 1-[4-[(2-aminoéthyl) amino]-N2-(12-méthyl-1-oxotétradécyl)-L-ornithine] 4-[4-(4-hydroxyphényl)-L-thréonine]-5-L-sérine échinocandine B (isomère A et isomère B).

A la solution de 300 mg de la préparation 1 dans 6 ml de méthanol en présence de 375 µl d'éthylènediamine est ajoutée 63 ml d'une solution 1M d'acide chlorhydrique dans le méthanol. Après 15 minutes d'agitation, on ajoute 126 mg de cyanoborohydrure de sodium (NaBH₃CN). On maintient le milieu réactionnel sous agitation pendant 5 heures. On filtre et amène à sec, les produits étant purifiés par HPLC (C₁₈) en éluant avec le mélange CH₃CN - H₂O - TFA (40-60-0,02) . On obtient ainsi les produits recherchés.
Spectre de masse :
MH⁺ = 1018
MNa⁺ = 1040.

### EXEMPLE 10 : (E) 1-[4- [(2-bromoéthoxy) imino]-N2-(12-méthyl-1-oxotétradécyl)-L-ornithine] 4-[4-(4-hydroxyphényl)-L-thréonine]-5-L-sérine échinocandine B et isomère Z correspondant.

On ajoute 402 mg de bromhydrate de bromo-2-éthoxyamine dans une solution renfermant 710 mg de produit de la préparation 1 et 28 ml d'éthanol absolu. On porte le mélange au reflux pendant 55 minutes. On concentre sous pression réduite. On purifie le produit obtenu par flash chromatographie sur silice en éluant avec le mélange chlorure de méthylène-méthanol (9-1). On obtient les produits recherchés isomère A : Rf = 0,54, isomère B : Rf = 0,47.
Spectre de masse :
MH⁺ = 1095
MNa⁺ = 1117

### EXEMPLE 11 . (±) Trifluoroacétate de 1-[4-[(aminoiminométhyl) hydrazono]-N2-(12-méthyl-1-oxotétradécyl)-L-ornithine] 4-[4-(4-hydroxyphényl)-L-thréonine]-5-L-sérine échinocandine B.

On ajoute 162 mg de chlorhydrate d'aminoguanidine dans une solution renfermant 260 mg du produit de la préparation 1 et 10 ml de n-butanol. On porte le milieu réactionnel au reflux pendant 2 heures 30 minutes. On concentre sous pression réduite. On purifie le produit obtenu par HPLC semi-préparative. On obtient 225 mg de produit en mélange d'isomères 50/50.
Spectre de masse :
MH⁺ = 1030
MNa⁺ = 1052.

### EXEMPLE 12 : (Z) Trifluoroacétate de 1-[4-[[2-(diméthylamino) éthoxyimino]-N2-(12-méthyl-1-oxotétradécyl)-L-ornithine] 4-[4-(4-hydroxyphényl)-L-thréonine]-5-L-sérine échinocandine B et isomère E correspondant.

On introduit 80,5 mg de produit de l'exemple 10 dans 32 ml d'une solution éthanolique de diméthylamine. On porte le milieu réactionnel au reflux pendant 45 minutes. On concentre. On purifie le produit obtenu par HPLC (C₁₈) (CH₃CN - H₂O - TFA 60-40-0,02). On obtient ainsi les produits recherchés.
Spectre de masse :
MH⁺ = 1060

### EXEMPLE 13 : (E) Trifluoroacétate de 1-[4-[(2-aminoéthoxy) imino]-N2-(12-méthyl-1-oxotétradécyl)-L-ornithine] 4-[4-(4-hydroxyphényl)-L-thréonine]-5-L-sérine échinocandine B et isomère Z correspondant.

On introduit 50 mg de produit de l'exemple 10 dans de l'ammoniac. On agite sous pression pendant 16 heures en laissant revenir à température ambiante. On reprend le milieu réactionnel dans le mélange CH₃CN-H₂O (45-55) pour être purifié par HPLC (C₁₈). On obtient les produits recherchés.
Spectre de masse :
MH⁺ = 1032.

### Préparation 2 : "nucléus" de déoxymulundocandine

On dissout 2 g de déoxymulundocandine dans 20 ml de DMSO. On verse cette solution dans une suspension renfermant 120 g d'Actinoplanes utahensis FH2264 dans 870 ml d'un tampon KH2PO4, K2HPO4 (pH : 6.8). On maintient le mélange réactionnel sous agitation pendant 70 heures à 30°C. On filtre. On lave le mycelium avec le tampon de phosphate (pH : 6.8). On réunit les liquides de lavage et le filtrat. On chromatographie le produit obtenu sur une résine DIAION HP 20.et obtient un produit que l'on utilise tel quel ci-après.

### EXEMPLE 14

### Trifluoroacétate de 1-[4-[(2-aminoéthyl) amino]-N2-[[4'-(octyloxy) [1,1'-biphényl]-4-yl]carbonyl]-L-ornithine]-4- [4-(4-hydroxyphényl)-L-thréonine]-5-L-sérine échinocandine B (isomère A)

### Stade A : 1-[(4R,5R)-4,5-dihydroxy-N2-[[4'-(octyloxy)[1,1'-biphényl]-4-yl]carbonyl]-L-ornithine]-4-[4-(4-hydroxyphényl)-L-thréonine]-5-L-sérine échinocandine B

### 1- Préparation de l'ester

On ajoute 632 g de 2,3,4,5,6 pentafluorophénol dans 695 mg de N,N'-dicyclohexylcarbodiimide à 1 g d'acide 4'-octyloxy-[1,1'-biphényl]4-carboxylique dans 22 ml de tétrahydrofurane, agite 22 heures à température ambiante, filtre, élimine les solvants sous pression réduite, reprend le résidu dans l'éther, agite à 35°C environ, filtre évapore le solvant , sèche et récupère 1,46 g de produit attendu, utilisé tel quel.

### 2- Couplage

On introduit 677 mg de « nucléus » de déoxymulundocandine obtenu à la préparation 2, dans 16 ml de DMF. On agite la solution obtenue pendant 5 minutes et ajoute 793 mg de 4'-(octyloxy)-[1,1'-biphényl]-4-carboxylate de pentafluorophényle obtenu ci-dessus.
On maintient le mélange réactionnel sous agitation et atmosphère d'azote pendant 24 heures. On filtre et concentre. On reprend le résidu à l'éther, triture, maintient 25 minutes sous agitation, essore, lave à l'éther éthylique, chromatographie sur silice en éluant avec le mélange chlorure de méthylène, méthanol, eau (86/13/1) puis (80/20/1). On obtient ainsi le produit recherché. Rendement 73%.

### Stade B : 1-[N2-[[4'-(octyloxy)-[1,1'-biphényl]-4-yl] carbonyl]-4-oxo-L-ornithine]-4-[4-(4-hydroxyphényl)-L-thréonine]-5-L-serine-échinocandine B

On ajoute 311 *µ*l d'iodure de triméthylsilyle dans une suspension renfermant 809 mg de produit du stade A et 19 ml d'acétonitrile. On maintient le mélange réactionnel sous agitation pendant 15 minutes à 60°C et sous atmosphère d'azote. On verse le mélange dans une solution saturée en thiosulfate de sodium. On évapore et chromatographie sur silice le résidu obtenu, en éluant avec le mélange chlorure de méthylène-méthanol eau 86/13/1. On obtient le produit recherché. Rendement 55%.

### Stade C : Trifluoroacétate de 1-[4-[(2-aminoéthyl) amino]-N2-[[4'-(octyloxy) [1,1'-biphényl]-4-yl]carbonyl]-L-ornithine]-4-[4-(4-hydroxyphényl)-L-thréonine]-5-L-sérine échinocandine B (isomère A)

On ajoute 560 *µ*l d'acide acétique dans une solution renfermant 900 mg de produit du stade précédent 16 ml de méthanol et 250 *µ*l d'éthylène diamine. On agite pendant 15 minutes et ajoute 64 mg de cyanoborohydrure de sodium. On agite pendant 18 heures. On filtre et concentre. On reprend le résidu dans le minimum d'eau, triture, essore et purifie par HPLC préparative en éluant avec le mélange CH₃CN/H₂O/TFA/ (55-45-0,2). On obtient le produit recherché. Rendement 26 %.
**Spectre RMN CDCl**_{**3**}
9.07 (m large) 1H ; 8.48 (dl, J=8) 1H ; 8.00 (dl, J=8) 2H ; 7.96 (dl, J=8.5) 2H ; 7.71 (dl, J=8.5) 2H ; 7.64 (dl, J=8.5) 2H ; 7.60 (dl, J=9) 1H ; 7.37 (dl, J=9.5) 1H ; 7.02 (dl, J=8.5) 2H ; 6.97 (dl, J=8.5) 2H ; 6.65 (dl, J=8.5) 2H ; 4.90 (m) 1H ; 4.77 (m) 1H ; 4.66 (m) 1H ; 4.45 (m) 1H ; 4.42 (m) 1H ; 4.39 (m) 1H ; 4.34 (s1) 1H ; 4.26 (m) 1H ; 4.22 (m) 1H ; 4.08 (m) 1H ; 4.01 (t,J=6.5) 2H ; 3.88 (m) 3H ; 3.70 (m) 2H ; 3.51 (m) 2H ; 3.48 (m) 1H ; 3.31 (m) 2H ; 3.28 (m) 1H ; 3.16 (m) 2H ; 2.53 (dd, J=6 et 13.5) 1H ; 2.44 (dd, J=7.5 et 13.5) 1H ; 2.27 (m) 1H ; 2.25 (m) 1H ; 2.15 (m) 2H ; 1.94 (m) 1H ; 1.74 (m) 2H ; 1.44 (m) 2H ; 1.22 à 1.40 (m) 8H ; 1.13 (d, J=6) 3H ; 0.99 (d, J=6.5) 3H ; 0.88 (t, J=7) 3H.

### EXEMPLE 15 : 1- [4-[(aminoiminométhyl)hydrano]-N2-[[4-[4-[4-(pentyloxy)-phényl]-1-piperazinyl]phényl]carbonyl]-L-ornithine] -4- [4- (4-hydroxyphényl) -L-thréonine] -5-L-sérine-échinocandine B

### Stade A : 1- [(4R,5R)-4,5-dihydroxy-N2-[[4-[4- [4- (pentyloxy) phényl]-1-pipérazinyl]phényl]carbonyl]-L-ornithine]-4-[4-(4-hydroxy-phényl)-L-thréonine]-5-L-sérine échinocandine B

### 1- Préparation de l'ester

On ajoute 55 mg de pentafluorophénol et 61 mg de N,N' dicyclohexyl carbodiimide dans un mélange de 100 mg d'acide [4-[4-[4-(pentyloxy)phényl]-1-pipérazinyl]phényl]carboxylique et 3 ml de tétrahydrofuranne. On agite le mélange réactionnel à 20°C pendant 16 heures, filtre, lave au THF et concentre à sec. On reprend dans l'éther diéthylique, filtre, lave et concentre. On obtient 71 mg de produit.

### 2- Couplage

On agite à 20°C pendant une nuit une suspension renfermant 71 mg de l'ester ci-dessus, 70 mg du « nucléus » de déoxymulundocandine obtenu comme à la préparation 2, 2,5 ml de DMF en présence de siliporite activé 4Å. On concentre, reprend à l'éther le produit obtenu et filtre. On obtient un produit que l'on chromatographie sur silice en éluant avec le mélange acétonitrile/eau/acide trifluoroacétique (60-40-0,02). On obtient ainsi 30 mg de produit recherché.

### Stade B : 1-[N2-[[4-[4-[4-(pentyloxy)phényl] -1-pipérazinyl]-phényl]-carbonyl]4-oxo-L-ornithine]-4-[4-(4-hydroxyphényl)-L-thréonine]-5-L-sérine échinocandine B

### 1- Préparation de l'ester

On chauffe à 55°C un mélange de 1 g de produit du stade A, 25 ml d'acétonitrile, en présence de siliporite active 4Å. On ajoute 430 ml d'iodure de triméthylsilane. On agite pendant 45 minutes puis ajoute 150 *µ*l d'une solution aqueuse de thiosulfate de sodium à 30%. On agite 40 minutes à 20°C et concentre. On reprend l'extrait sec dans l'eau, agite 1 heure à 20°C essore et lave. On obtient un produit que l'on chromatographie sur silice en éluant avec le mélange chlorure de méthylène-méthanol-eau (86/13/1). On obtient 497 mg de produit recherché. Rendement 42%.

### Stade C : 1-[4-[(aminoiminométhyl)hydrazono]-N2-[[4-[4-[4-(pentyloxy)-phényl]-1-piperazinyl]phényl]carbonyl]-L-ornithine]-4-[4-(4-hydroxyphényl)-L-thréonine]-5-L-sérine-échinocandine B

On chauffe à 130°C pendant 3 heures une suspension renfermant 400 mg de produit du stade B, 4,8 ml de n-butanol et 221 mg de chlorhydrate d'aminoguanidine. On concentre et obtient 705 mg d'un produit que l'on chromatographie sur silice en éluant avec le mélange chlorure de méthylène méthanol 85/15, puis par HPCL semi-préparative (kromasil C18)avec un mélange acétonitrile/eau/acide trifluoroacétique (40.60.0,02). On obtient ainsi 64 mg de produit recherché.
**Spectre RMN CDCl**_{**3**}
10.75 (s ) 0.66H ; 10.45 ( s ) 0.33H ; 8.39 ( d, J=8 ) 0.33H ; 8.34 ( m ) 1H ; 8.10 ( d, J=7.5 ) 0.66H ; 8.08 ( d, J=8 ) 0.33H ; 7.99 ( d, J=8.5 ) 0.66H ; 7.74 ( d, J=8.5 ) 1.33H ; 7.71 ( d, J=8.5 ) 0.66H ; 7.60 ( d, J=8.5 ) 0.66H ; 7.50 ( m ) 1.33H ; 7.00 ( m ) 6H ;6.86 ( d, J=8.5) 2H ; 6.65 ( d, J=8 ) 2H ; 5.08 ( dt, J=2 et 11.5 ) 0.66H ; 4.94 ( m ) 1H ; 4.88 ( m ) 0.33H ; 4.75 ( dm, J-8 ) 0.33H ; 4.67 ( dd, J=3 et 7.5 ) 0.66H ;4.43 ( m ) 1H ; 4.38 ( m ) 1.66H ;4.33 ( m ) 0.66H ;4.26 à 4.20 ( massif ) 2.33H ; 4.12 ( d, J=9 ) 0.66H ;4.00 à 3.68 ( massif ) 7.33H ; 3.90 ( t,J=7 ) 2H ;3.62 ( d,J=12 ) 0.33H ;3.43 (slarge) 2H ;3.30 à 3.20 (m ) 1H ; 3.20 ( slarge ) 2H ;2.91 ( d,J=14 )0.66H ;2.86 ( m ) 0.33H ; 2.76 ( m ) 0.33H ; 2.63 ( dd, J=14 et 12.5 ) 0.66H ;2.52 ( dt, J=6 et 13 ) 1H ; 2.44 ( dd, J=8 et 13 ) 1H ;2.35 5 ( m ) 0.33H ; 2.25 ( m ) 1.66H ;1.93 (tlarge,J=13 ) 1H ; 1.69 ( m ) 2H ; 1.42 à 1.30 ( massif ) 4H ;1.15 ( d, J=6 ) 1.98H ;1.10 ( ,J=6 ) 0.99H ; 0.98 ( d, J=6.5 ) 3H ; 0.90 ( t, J=7 ) 3H.

### EXEMPLE 16 : 1-[4-[(2-aminoéthyl)amino]-N2-[4-[4"-(pentyloxy) [1,1' : 4',1" - terphényl]-4-yl]carbonyl]-L-ornithine]-4-[4-(4-hydroxyphényl)-L-thréonine]-5-L-sérine-échinocandine B (isomère A et isomère B).

En opérant comme précédemment, à partir du « nucléus » de déoxy-mulundocandine préparé comme indiqué à la préparation 2 en obtenant comme produit intermédiaire le 1-[(4R,5R)-4,5-dihydroxy-N2-[[4''-(pentyloxy)[1,1' : 4', 1"-terphényl]-4-yl]carbonyl]-L-ornithine]-4-[4-(4-hydroxyphényl)-L-thréonine] -5-L-sérine-échinocandine B et le dérivé 4-oxo correspondant, on a obtenu le produit recherché.
**Spectre RMN CDCl**_{**3**} **ppm**
9.00 (large) 1H ; 8.37 (dl, J=8.5) 1H; 8.28 (m) 1H ; 8.10 (dl, J=6) 1H ; 8.02 (dl, J=8) 2H ; 7.82 (m) 4H ; 7.73 (dl, J=8) 2H; 7.66 (dl, J=8) 2H; 7.38 (dl, J=9) 1H ; 7.32 (dl, J=9) 1H ; 7.03 (dl, J=8.5) 2H ; 6.96 (dl, J=8) 2H ; 6.66 (dl, J=8) 2H ; 5.03 (m) 1H ; 4.84 (m) 1H ; 4.67 (m) 1H ; 4.45 (m) 2H ; 4.36 (dd, J=7.5 et 10.5) 1H ; 4.23 (m) 2H ; 4.18 (sl) 1H ; 4.04 (m) 1H ; 4.02 (t, J=6.5) 2H; 4.00 (m) 1H; 3.87 (dl, J=9.5) 1H; 3.76 (m) 1H ; 3.72 (m) 2H ; 3.55 (m) 1H ; 3.44 (m) 1H ; 3.35 (m) 2H ; 3.30 (m) 1H ; 3.19 (m) 2H ; 3.12 (m) 1H ; 2.53 (m) 1H ; 2.45 (m) 1H; 2.12 à 2.30 (m) 3H ; 1.90 à 2.05 (m) 2H ; 1.74 (m) 2H ; 1.30 à 1.55 (m) 4H ; 1.20 (d, J=5.5) 3H ; 0.96 (d, J=6.5) 3H ; 0.91 (t, J=7) 3H.

### EXEMPLE: Composition pharmaceutique :

On a préparé des comprimés renfermant :
- Produit de l'exemple 14........................ 150 mg
- Excipient q.s.p............................. 1 g
(Détail de l'excipient : amidon, talc, stéarate de magnésium).

### ETUDE PHARMACOLOGIQUE

### A - Inhibition de la glucane synthase de Candida albicans.

On purifie des membranes de Candida albicans selon le procédé décrit par Tang et al Antimicrob. Agents Chemother 35, 99-103, 1991. 22,5 *µ*g de protéines membranaires sont incubées dans un mélange de 2Mm de 14C-UDP glucose (activité spécifique = 0,34 mCi./mmol, 50 *µ*g d'α-amylase, 1Mm de dithiotreitol (DTT), 1Mm EDTA, 100Mm NaF, 7 *µ*M de GTP-γ-S, 1M de sucrose et 50Mm DE Tris-HCL (pH 7,8) dans un volume de 100*µ*l. Le milieu est incubé à 25°C pendant 1 heure et la réaction terminée par addition de TCA à une concentration finale de 5%. Le mélange réactionnel est transféré sur un filtre de fibre de verre pré-humidifié. Le filtre est lavé, séché et sa radioactivité est comptée.
La mulundocandine est utilisé comme contrôle positif.
Le contrôle du véhicule est effectué avec la même quantité de DMSO 1%. Les résultats obtenus montrent que les produits de l'invention présentent sur ce test une bonne activité en particulier les produits des exemples 9, 11 et 14.

### B - activité sur l'enzyme d'Aspergillus fumigatus.

L'enzyme est préparée selon le procédé de Beaulieu et al. (Antimicrob. Agents Chenother 38, 937-944, 1994.
Le protocole utilisé est identique au protocole décrit ci-dessus pour l'enzyme de Candida albicans sauf que l'on n'utilise pas de dithiotreitol dans le mélange réactionnel.

Les produits présentent sur ce test une bonne activité.

## Revendications

1. Sous toutes les formes d'isomères possibles ainsi que leurs mélanges, les composés de formule (I) : 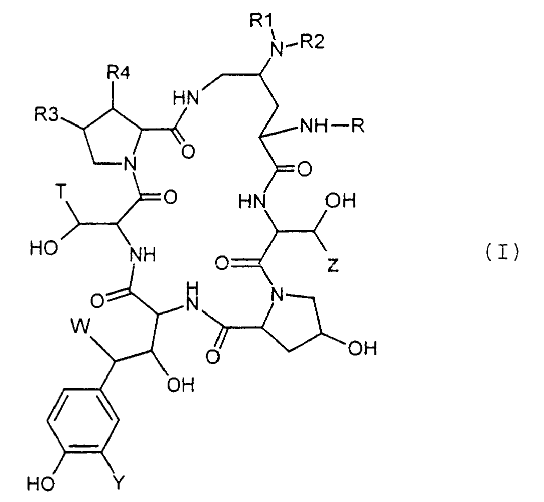 dans lesquels
ou bien R1 et R2 identiques ou différents l'un de l'autre, représentent un atome d'hydrogène, un radical hydroxyle, un radical alkyle renfermant jusqu'à 8 atomes de carbone linéaire, ramifié ou cyclique, éventuellement interrompu par un atome d'oxygène éventuellement substitué par un atome d'halogène, un radical OH, un radical 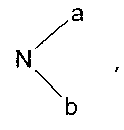 a et b identiques ou différents l'un de l'autre,
représentant un atome d'hydrogène ou un radical alkyle renfermant jusqu'à 8 atomes de carbone, a et b pouvant éventuellement former avec l'atome d'azote un hétérocycle renfermant éventuellement un ou plusieurs hétéroatomes supplémentaires,
- ou bien R1 forme avec l'atome de carbone endocyclique portant le radical 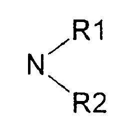 une double liaison et ou bien R2 représente un radical XRₐ, X représentant un atome d'oxygène ou un radical NH ou N-alkyle renfermant jusqu'à 8 atomes de carbone et Rₐ représente un atome d'hydrogène, un radical alkyle linéaire, ramifié ou cyclique renfermant jusqu'à 8 atomes de carbone éventuellement substitué par un ou plusieurs atomes d'halogène, par un ou plusieurs radicaux OH, CO₂H, CO₂alc,
par un radical , 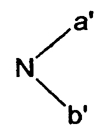 a' et b' représentant un atome d'hydrogène, un radical alkyle renfermant jusqu'à 8 atomes de carbone, a' et b' pouvant former un hétérocycle renfermant éventuellement un ou plusieurs hétéroatomes supplémentaires et/ou par un hétérocycle renfermant un ou plusieurs hétéroatomes ou R2 représente un radical 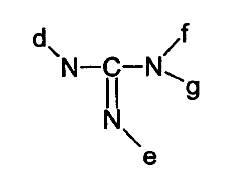
dans lequel d, e, f et g représentent un atome d'hydrogène ou un radical alkyle renfermant jusqu'à 8 atomes de carbone, f et g pouvant en outre représenter un radical acyle renfermant jusqu'à 8 atomes de carbone, e et f pouvant également former un cycle renfermant éventuellement un ou plusieurs hétéro-atomes,
R3 représente un atome d'hydrogène, un radical méthyle ou hydroxyle
R4 représente un atome d'hydrogène ou un radical hydroxyle
R représente une chaîne linéaire ou ramifiée ou cyclique renfermant jusqu'à 30 atomes de carbone, renfermant éventuellement, un ou plusieurs hétéroatomes, un ou plusieurs hétérocycles ou un radical acyle linaire, ramifié ou cyclique renfermant jusqu'à 30 atomes de carbone renfermant éventuellement un ou plusieurs hétéroatomes et/ou un ou plusieurs hétérocycles,
T représente un atome d'hydrogène, un radical méthyle, un radical CH₂CONH₂, CH₂C≡N, un radical (CH₂)₂NH₂ ou (CH₂)₂NalC₂⁺X⁻,
X étant un atome d'halogène et alc un radical alkyle renfermant jusqu'à 8 atomes de carbone,
Y représente un atome d'hydrogène, un radical hydroxyle ou un atome d'halogène ou un radical OSO3H ou l'un des sels de ce radical,
W représente un atome d'hydrogène ou un radical OH,
Z représente un atome d'hydrogène ou un radical méthyle, ainsi que les sels d'addition avec les acides des produits de formule (I) .

2. Les composés de formule (I) définis à la revendication 1 dans lesquels T représente un atome d'hydrogène.

3. Les composés de formule (I) définis à la revendication 1 ou 2 dans lesquels W représente un atome d'hydrogène.

4. Les composés de formule (I) définis à l'une quelconque des revendications 1 à 3, dans lesquels Z représente un radical méthyle.

5. Les composés de formule (I) définis à l'une quelconque des revendications 1 à 4 dans lesquels Y représente un atome d'hydrogène.

6. Les composés de formule (I) définis à l'une quelconque des revendications 1 à 5 dans lesquels R3 représente un radical méthyle.

7. Les composés de formule définis à l'une quelconque des revendications 1 à 6 dans lesquels R4 représente un radical hydroxyle.

8. Les composés de formule (I) définis à l'une quelconque des revendications 1 à 7 dans lesquels R représente un radical

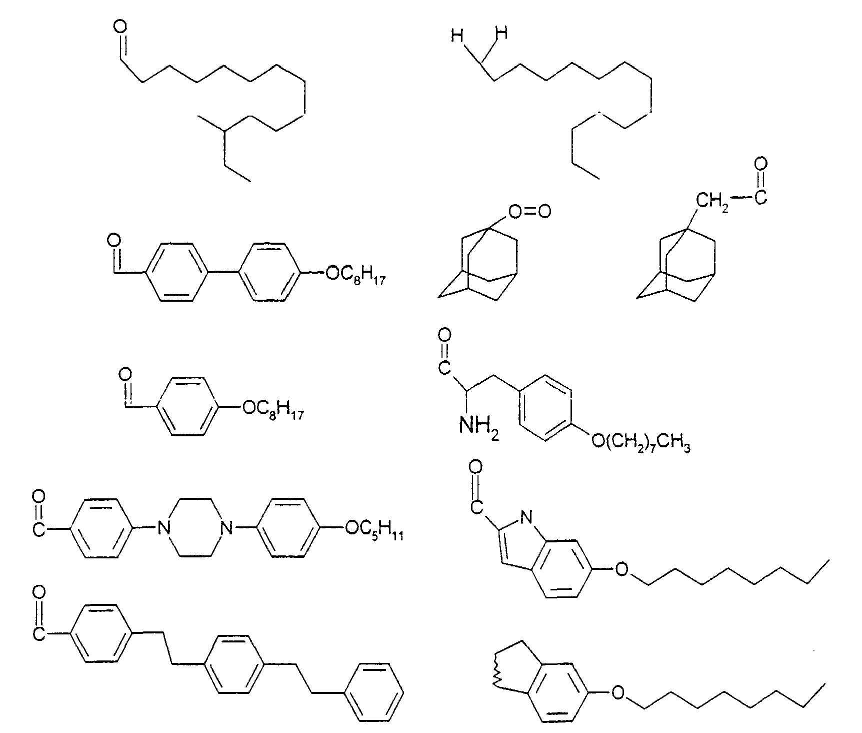

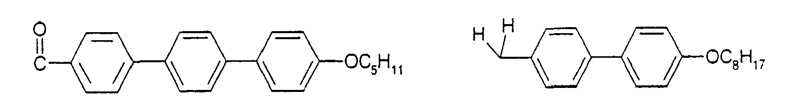

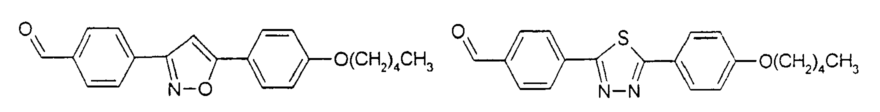

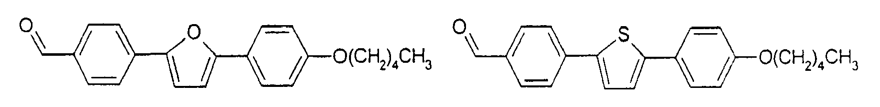

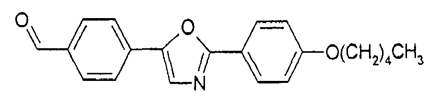

9. Les composés de formule (I) définis à la revendication 8, dans lesquels R représente une chaîne

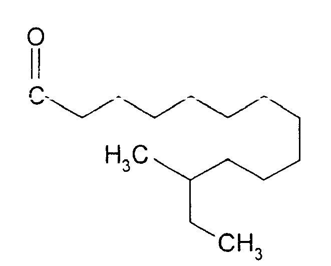

10. Les composés de formule (I) définis à la revendication 8, dans lesquels R représente une chaîne

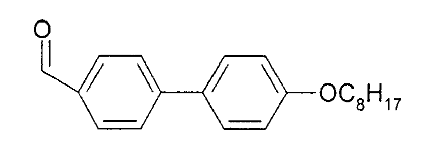

11. Les composés de formule (I) définis à l'une des revendications 1 à 10, dans lesquels R1 forme avec l'atome de carbone endocyclique portant le radical NR1R2, une double liaison.

12. Les composés de formule (I) définis à la revendication 11, dans lesquels R2 représente le radical
O(CH₂) ₙNY'₂
dans lequel n représente un nombre entier compris entre 1 et 8 et Y' représente un atome d'hydrogène ou un radical alkyle renfermant jusqu'à 8 atomes de carbone.

13. Les composés de formule (I) définis à la revendication 12, dans lesquels n représente le nombre 2.

14. Les composés de formule (I) définis à la revendication 8, dans lesquels R2 représente un radical

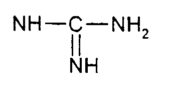

15. Les composés de formule (I) définis à l'une des revendications 1 à 10, dans lesquels R2 représente un radical
(CH₂)_{P}NY"₂
dans lequel Y" représente un atome d'hydrogène ou un radical alkyle renfermant jusqu'à 8 atomes de carbone et p représente un nombre entier variant de 1 à 8.

16. Les composés de formule (I) définis à l'une quelconque des revendications 1 à 10 et 15, dans lesquels R1 représente un atome d'hydrogène.

17. Les composés de formule (I) définis à la revendication 15, dans lesquels p représente le nombre 2.

18. Les composés de formule (I) définis à la revendication 1, dont les noms suivent :
- la 1-[(S)-N2-(12-méthyl-1-oxotétradécyl) 4-[[(3-pipéri-dinyl) oxy] imino]-L-ornithine] 4-[4-(4-hydroxyphényl)-L-thréonine]-5-L-sérine échinocandine B.
- la 1-[4-[(2-aminoéthyl) amino]-N2-(12-méthyl-1-oxotétra-décyl)-L-ornithine] 4-[4-(4-hydroxyphényl)-L-thréonine]-5-L-sérine échinocandine B (isomère A et isomère B).
- la 1-[4-[(aminoiminométhyl) hydrazono]-N2-(12-méthyl-1-oxotétradécyl)-L-ornithine] 4-[4-(4-hydroxyphényl)-L-thréonine]-5-L-sérine échinocandine B.
- la 1-[4-[(2-aminoéthoxy) imino]-N2-(12-méthyl-1-oxotétradécyl)-L-ornithine] 4-[4-(4-hydroxyphényl)-L-thréonine]-5-L-sérine échinocandine B et isomère Z correspondant,
- la 1-[4-[(2-aminoéthyl)amino]-N2- [[4'-(octyloxy) [1,1'-biphényl]-4-yl]carbonyl]-L-ornithine]-4-[4-(4-hydroxyphényl)-L-thréonine]-5-L-serine-echinocandine B (isomère A)
ainsi que leurs sels d'addition avec les acides.

19. Un composé de formule (I) telle que définie à la revendication 1 dans laquelle T, W, Y et R₁ représentent un atome d'hydrogène, Z et R₃ représentent un radical méthyle, R₂ représente un radical CH₂-CH₂-NH₂, R₄ représente un radical hydroxyle, R représente un groupement 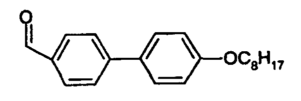 ainsi que ses sels d'addition avec les acides

20. Procédé de préparation des composés de formule (I) définis à l'une quelconque des revendications 1 à 18, **caractérisé en ce que** l'on soumet un composé de formule (II) 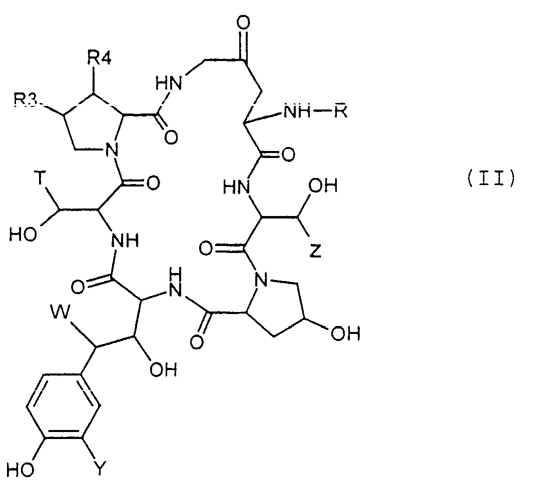 dans laquelle R, R3, R4, T, Y, W et Z conservent leur signification précédente, à l'action d'une amine ou d'un dérivé d'amine susceptible d'introduire le radical 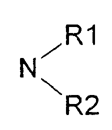 dans lequel R1 et R2 conservent leur signification précédente et si désiré à l'action d'un agent de réduction
et/ou d'un agent de fonctionnalisation de l'amine,
et/ou d'un acide pour former le sel du produit obtenu,
et/ou d'un agent de séparation des différents isomères obtenus,
et obtient ainsi le composé de formule (I) recherché 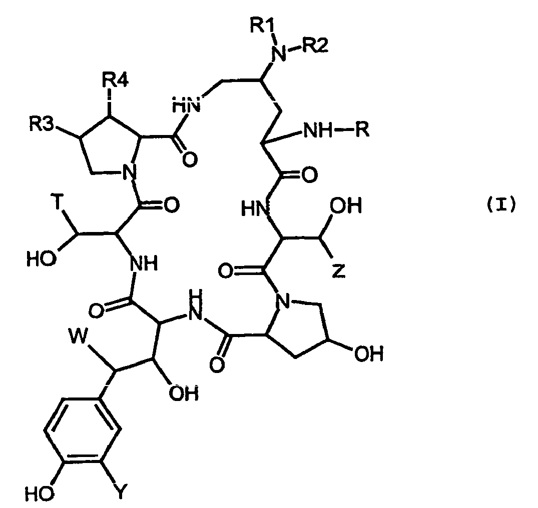 dans laquelle R1, R2, R3, R4, T, Y, W, R et Z conservent leur signification précédente puis soumet, si désiré le composé de formule (I) à l'action d'un acide pour en former le sel et sépare si désiré les différents isomères obtenus.

21. A titre de produits chimiques nouveaux, les composés de formule (II) définis à la revendication 19.

22. A titre de produit chimique nouveau défini à la revendication 20, le composé de formule (II) dont les noms suivent :
le 1([-4-oxo-N2-(12-méthyl-1-oxotétradécyl) L-ornithine] 4- [4- (4-hydroxyphényl)-L-thréonine]-5-L-sérine-échinocandine B.
1-[N2-[[4'-(octyloxy)-[1,1'-biphényl]-4-yl]carbonyl]-4-oxo-L-ornithine]-4-[4-(4-hydroxyphényl)-L-thréonine]-5-L-serine-échinocandine B
1-[N2-[[4-[4-[4-(pentyloxy)phényl]-1-pipérazinyl]-phényl]-carbonyl]4-oxo-L-ornithine]-4-[4-(4-hydroxyphényl)-L-thréonine]-5-L-sérine échinocandine B

23. Procédé selon la revendication 19 **caractérisé en ce que** l'on soumet un composé de formule (III) 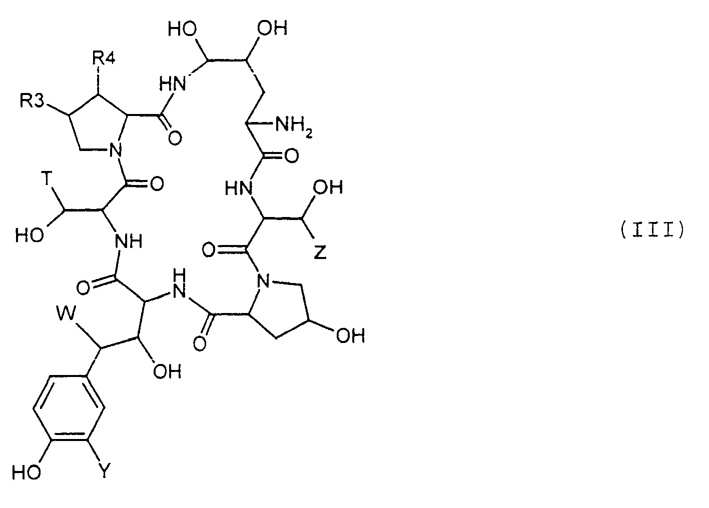 dans laquelle les différents substituants conservent leur signification précédente à l'action d'un agent capable de remplacer NH₂ par NHR, R conservant sa signification précédente pour obtenir le composé de formule (IV) 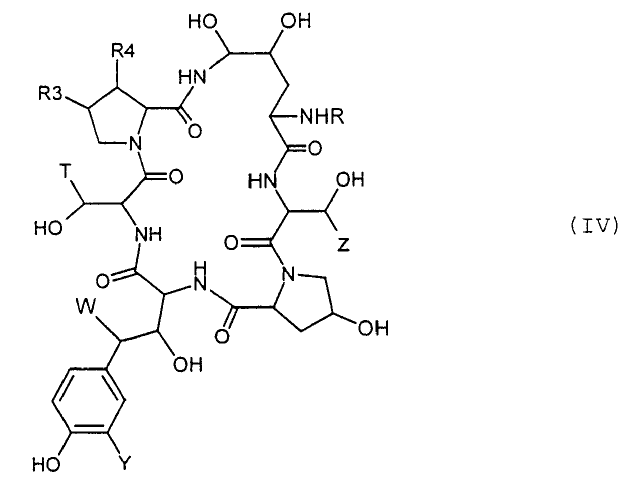 que l'on soumet à l'action de l'iodure de triméthylsilyle pour obtenir le composé de formule (II) correspondant

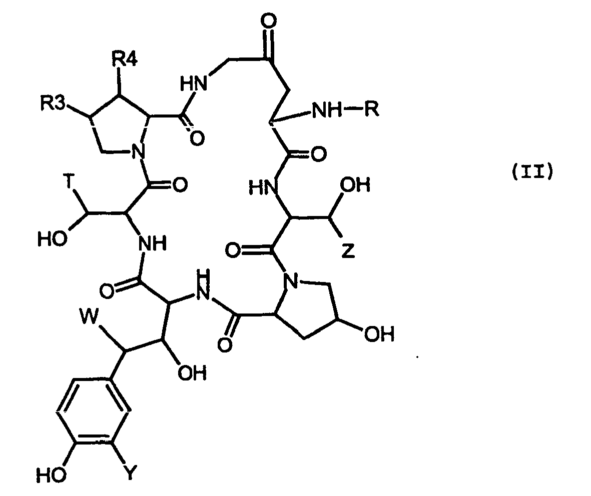

24. A titre de composés antifongiques, les composés de formule (I) définis à l'une quelconque des revendications 1 à 19, ainsi que leurs sels d'addition avec les acides.

25. Les compositions pharmaceutiques renfermant à titre de médicament au moins un composé de formule (I) défini à l'une quelconque des revendications 1 à 19, ainsi que leurs sels d'addition avec les acides pharmaceutiquement acceptables.

## Patentansprüche

1. Verbindungen der Formel (I) in allen möglichen isomeren Formen sowie ihre Gemische: 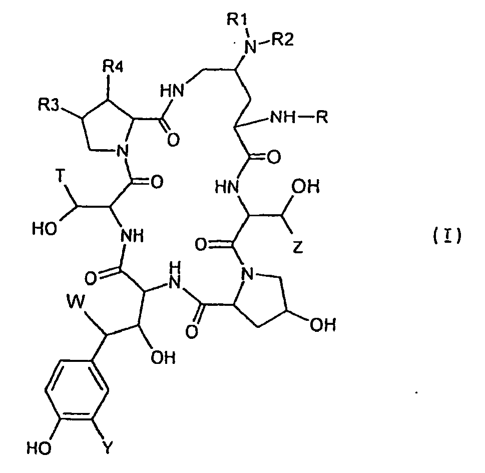 wobei
R1 und R2 entweder identisch oder voneinander verschieden sind, ein Wasserstoffatom, einen Hydroxylrest, einen Alkylrest darstellen, der bis zu 8 Kohlenstoffatome umfasst, linear, verzweigt oder cyclisch ist, gegebenenfalls durch ein Sauerstoffatom unterbrochen ist, gegebenenfalls mit einem Halogenatom, einem Hydroxylrest, einem Rest 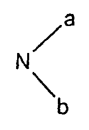 substituiert ist, wobei a und b identisch oder voneinander verschieden sind, ein Wasserstoffatom oder einen Alkylrest darstellen, der bis zu 8 Kohlenstoffatome umfasst, a und b gegebenenfalls mit dem Stickstoff einen Heterozyklus bilden, der gegebenenfalls ein oder mehrere zusätzliche Heteroatome umfasst;
oder R1 mit dem endocyclischen Kohlenstoffatom, das den Rest 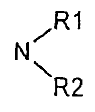 trägt, eine Doppelbindung bildet, oder R2 einen Rest XRₐ darstellt, wobei X ein Sauerstoff oder einen Rest NH oder N-Alkyl, der bis zu 8 Kohlenstoffatome umfasst, darstellt und Rₐ ein Wasserstoffatom, einen linearen, verzweigten oder cyclischen Alkylrest, der bis zu 8 Kohlenstoffatome umfasst, darstellt, der gegebenenfalls mit einem oder mehreren Halogenatomen, einem oder mehreren Resten OH, CO₂H, CO₂Alk, mit einem Rest 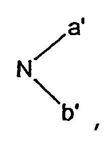 wobei a' und b' ein Wasserstoffatom, einen Alkylrest, der bis zu 8 Kohlenstoffatome umfasst, darstellen, a' und b' einen Heterozyklus bilden können, der gegebenenfalls ein oder mehrere zusätzliche Heteroatome umfasst und/oder mit einem Heterozyklus, der ein oder mehrere Heteroatome umfasst, substituiert ist; oder R2 einen Rest 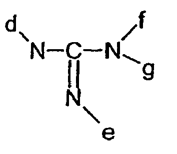 darstellt, wobei d, e, f und g ein Wasserstoffatom oder einen Alkylrest, der bis zu 8 Kohlenstoffatome umfasst, darstellen; f und g außerdem einen Acylrest darstellen können, der bis zu 8 Kohlenstoffatome umfasst, e und f auch einen Ring bilden können, der gegebenenfalls ein oder mehrere Heteroatome umfasst;
R3 ein Wasserstoffatom, einen Methyl- oder Hydroxylrest darstellt;
R4 ein Wasserstoffatom oder einen Hydroxylrest darstellt;
R eine lineare oder verzweigte oder cyclische Kette, die bis zu 30 Kohlenstoffatome umfasst, darstellt, umfassend gegebenenfalls ein oder mehrere Heteroatome, einen oder mehrere Heterozyklen oder einen linearen, verzweigten oder cyclischen Acylrest, der bis zu 30 Kohlenstoffatome umfasst, gegebenenfalls ein oder mehrere Heteroatome und/oder einen oder mehrere Heterozyklen umfasst;
T ein Wasserstoffatom, einen Methylrest, einen Rest CH₂CONH₂, CH₂C=N, einen Rest (CH₂)₂NH₂ oder (CH₂)₂NAlk₂⁺X⁻ darstellt;
X ein Halogenatom ist und Alk ein Alkylrest ist, der bis zu 8 Kohlenstoffatome umfasst;
Y ein Wasserstoffatom, ein Hydroxylrest oder ein Halogenatom oder ein -OSO₃H-Rest oder ein Salz dieses Restes ist;
W ein Wasserstoffatom oder einen OH-Rest darstellt;
Z ein Wasserstoffatom oder einen Methylrest darstellt;
sowie die Additionssalze der Produkte der Formel (I) mit Säuren.

2. Verbindungen der Formel (I), die in Anspruch 1 definiert sind, wobei T ein Wasserstoffatom darstellt.

3. Verbindungen der Formel (I), die in Anspruch 1 oder 2 definiert sind, wobei W ein Wasserstoffatom darstellt.

4. Verbindungen der Formel (I), die in einem der Ansprüche 1 bis 3 definiert sind, wobei Z einen Methylrest darstellt.

5. Verbindungen der Formel (I), die in einem der Ansprüche 1 bis 4 definiert sind, wobei Y ein Wasserstoffatom darstellt.

6. Verbindungen der Formel (I), die in einem der Ansprüche 1 bis 5 definiert sind, wobei R3 einen Methylrest darstellt.

7. Verbindungen der Formel (I), die in einem der Ansprüche 1 bis 6 definiert sind, wobei R4 einen Hydroxylrest darstellt.

8. Verbindungen der Formel (I), die in einem der Ansprüche 1 bis 7 definiert sind, wobei R einen Rest 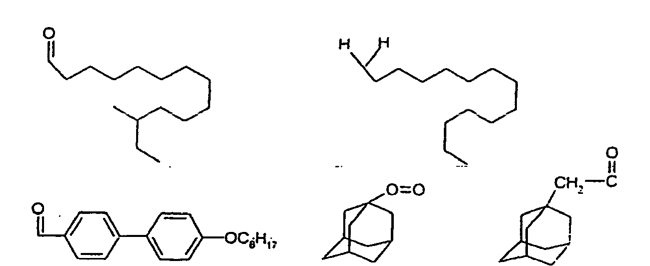 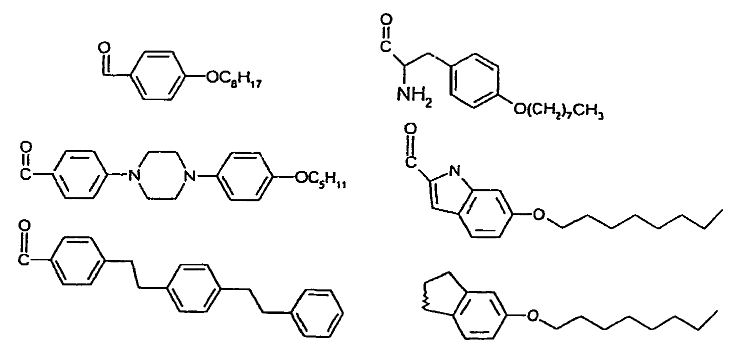 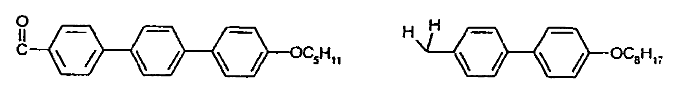 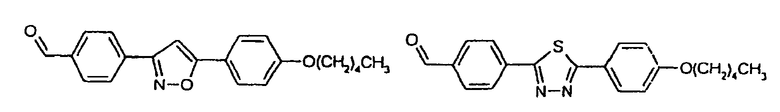 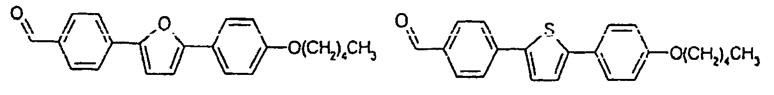 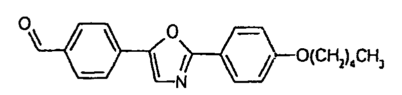 darstellt.

9. Verbindungen der Formel (I), die in Anspruch 8 definiert sind, in denen R eine Kette 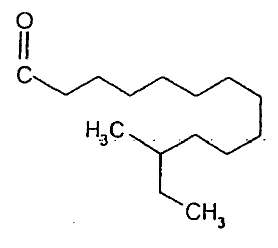 darstellt.

10. Verbindungen der Formel (I), die in Anspruch 8 definiert sind, in denen R eine Kette 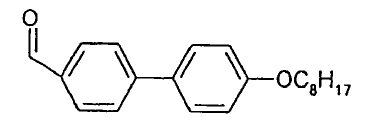 darstellt.

11. Verbindungen der Formel (I), die in einem der Ansprüche 1 bis 10 definiert sind, wobei R1 mit dem endocyclischen Kohlenstoffatom, das den Rest NR1R2 trägt, eine Doppelbindung bildet.

12. Verbindungen der Formel (I), die in Anspruch 11 definiert sind, wobei R2 den Rest
O(CH₂) ₙNY' ₂
darstellt, worin n eine ganze Zahl zwischen 1 und 8 darstellt und Y' ein Wasserstoffatom oder einen Alkylrest, der bis zu 8 Kohlenstoffatome umfasst, darstellt.

13. Verbindungen der Formel (I), die in Anspruch 12 definiert sind, wobei n die Zahl 2 darstellt.

14. Verbindungen der Formel (I), die in Anspruch 8 definiert sind, wobei R2 einen Rest 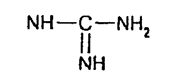 darstellt.

15. Verbindungen der Formel (I), die in einem der Ansprüche 1 bis 10 definiert sind, wobei R2 einen Rest
(CH₂)ₚNY"₂
darstellt, worin Y" ein Wasserstoffatom oder einen Alkylrest, der bis zu 8 Kohlenstoffatome umfasst, darstellt und p eine ganze Zahl zwischen 1 und 8 darstellt.

16. Verbindungen der Formel (I), die in einem der Ansprüche 1 bis 10 und 15 definiert sind, wobei R1 ein Wasserstoffatom darstellt.

17. Verbindungen der Formel (I), die in Anspruch 15 definiert sind, wobei p die Zahl 2 ist.

18. Verbindungen der Formel (I), die in Anspruch 1 definiert sind, mit den folgenden Namen:
- 1-[(S)-N2-(12-Methyl-1-oxotetradecyl)-4-[[(3-piperidinyl)oxy]imino]-L-ornithin]-4-[9-(4-hydroxyphenyl)-L-threonin]-5-L-serin-echinocandin B;
- 1-[4-[(2-Aminoethyl)amino]-N2-(12-methyl-1-oxotetradecyl)-L-ornithin]-4-[4-(4-hydroxyphenyl)-L-threonin]-5-L-serin-echinocandin B (Isomer A und Isomer B);
- 1-[4-[(Aminoiminomethyl)hydrazono]-N2-(12-methyl-1-oxotetradecyl)-L-ornithin]-4-[4-(4-hydroxyphenyl)-L-threonin]-5-L-serin-echinocandin B;
- 1-[4-[(2-Aminoethoxy)imino]-N2-(12-methyl-1-oxotetradecyl)-L-ornithin]-4-[4-(4-hydroxyphenyl)-L-threonin]-5-L-serin-echinocandin B und entsprechendes Z-Isomer,
- 1-[4-[(2-Aminoethyl)amino]-N2-[[4'-(octyloxy) [1,1'-biphenyl]-4-yl]carbonyl]-L-ornithin]-4-[4-(4-hydroxyphenyl)-L-threonin]-5-L-serin-echinocandin B (Isomer A)
sowie ihre Additionssalze mit Säuren.

19. Verbindung der Formel (I), wie sie in Anspruch 1 definiert ist, wobei T, W, Y und R₁ ein Wasserstoffatom darstellen, Z und R₃ einen Methylrest darstellen, R₂ einen Rest CH₂₋CH₂-NH₂ darstellt, R₄ einen Hydroxylrest darstellt, R eine Gruppierung 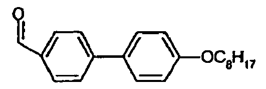 darstellt, sowie ihre Additionssalze mit Säuren.

20. Verfahren zur Herstellung der Verbindungen der Formel (I), die in einem der Ansprüche 1 bis 18 definiert sind, **dadurch gekennzeichnet , dass** man eine Verbindung der Formel (II) 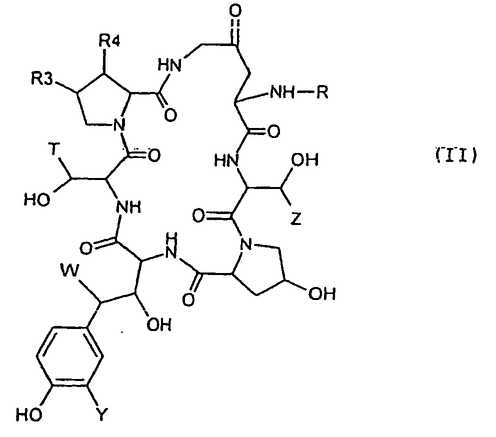 wobei R, R3, R4, T, Y, W und Z ihre vorstehend genannte Bedeutung behalten, der Wirkung eines Amins oder eines Aminderivats, das fähig ist, den Rest 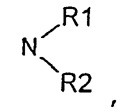 worin R1 und R2 ihre vorstehende Bedeutung behalten, einzuführen, unterwirft und gegebenenfalls der Wirkung eines Reduktionsmittels und/oder eines Funktionalisierungsmittels für das Amin und/oder einer Säure zur Bildung des Salzes des erhaltenen Produkts, und/oder einem Trennungsmittel für die verschiedenen erhaltenen Isomeren unterwirft und man so die gewünschte Verbindung der Formel (I) erhält 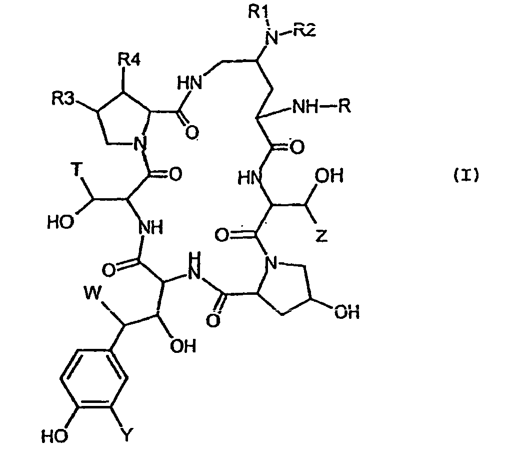 wobei R1, R2, R3, R4, T, Y, W, R und Z ihre vorstehende Bedeutung behalten; dann, wenn gewünscht, die Verbindung der Formel (I) der Wirkung einer Säure zur Bildung des Salzes derselben unterworfen wird und, wenn gewünscht, die verschiedenen erhaltenen Isomeren getrennt werden.

21. Verbindungen der Formel (II), die in Anspruch 19 definiert ist, als neue chemische Verbindungen.

22. Verbindung der Formel (II) mit den folgenden Namen:
1-([4-Oxo-N2-(12-methyl-1-oxotetradecyl)L-ornithin]-4-[4-(4-hydroxyphenyl)-L-threonin]-5-L-serin-echinocandin B;
1-[N2-[[4'-(Octyloxy)-[1,1'-biphenyl]-4-yl]carbonyl]-4-oxo-L-ornithin]-9-[4-(4-hydroxyphenyl)-L-threonin]-5-L-serin-echinocandin B;
1-[N2-[[4-[4-[4-(Pentyloxy)phenyl]-1-piperazinyl]-phenyl]-carbonyl]4-oxo-L-ornithin]-4-[4-(4-hydroxyphenyl)-L-threonin]-5-L-serin-echinocandin B
als neues chemisches Produkt, das in Anspruch 20 definiert ist.

23. Verfahren nach Anspruch 19, **dadurch** g e k e n n z e i c h - n e t , dass man eine Verbindung der Formel (III) 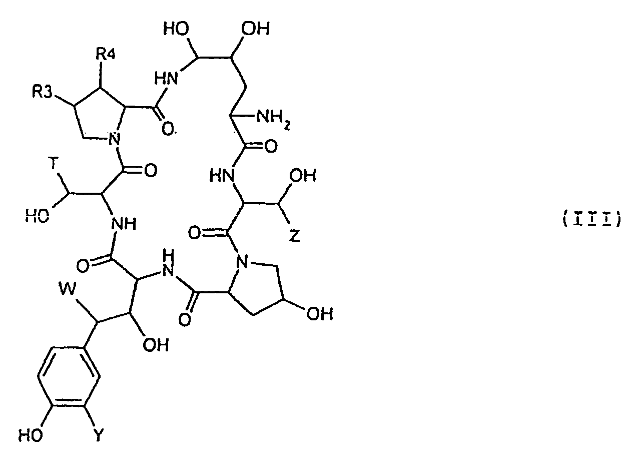 wobei die verschiedenen Substituenten ihre vorstehend genannte Bedeutung beibehalten, der Wirkung eines Agenses unterwirft, das fähig ist, NH₂ durch NHR zu ersetzen, wobei R seine vorstehend genannte Bedeutung behält, um die Verbindung der Formel (IV) zu erhalten: 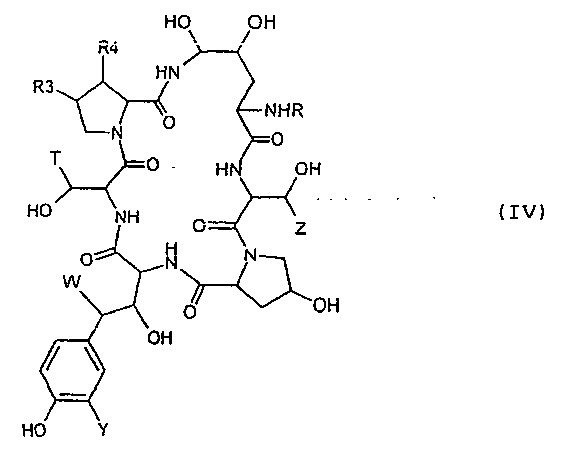 die man der Wirkung von Trimethylsilyliodid unterwirft, um die Verbindung der Formel (II) zu erhalten, die 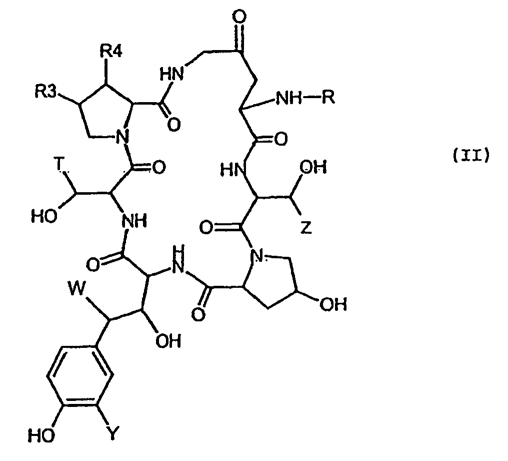 entspricht.

24. Verbindungen der Formel (I), die in einem der Ansprüche 1 bis 19 definiert sind, sowie ihre Additionssalze mit Säuren als antifungale Verbindungen.

25. Pharmazeutische Zusammensetzungen, die als Medikament wenigstens eine Verbindung der Formel (I), die in einem der Ansprüche 1 bis 19 definiert ist, sowie ihre Additionssalze mit pharmazeutisch annehmbaren Säuren umfassen.

## Claims

1. In all possible isomer forms as well as their mixtures, the compounds of formula (I): 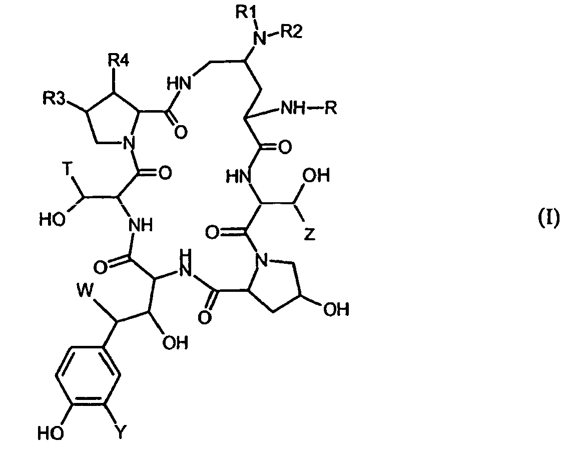 in which
either R1 and R2 identical or different, represent a hydrogen atom, a hydroxyl radical, a linear, branched or cyclic alkyl radical containing up to 8 carbon atoms, optionally interrupted by an oxygen atom optionally substituted by a halogen atom, an OH radical,
an 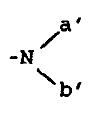 radical, a and b, identical or different, representing a hydrogen atom or an alkyl radical containing up to 8 carbon atoms, a and b optionally being able to form with the nitrogen atom a heterocycle optionally containing one or more additional heteroatoms,
R1 forms with the endocyclic carbon atom carrying the radical, a double bond 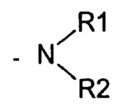 and either R2 represents an XRₐ radical, X representing an oxygen atom or an NH, or N-alkyl radical, alkyl containing up to 8 carbon atoms, and Rₐ represents a hydrogen atom, a linear, branched or cyclic alkyl radical containing up to 8 carbon atoms an 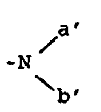 optionally substituted by one or more halogen atoms, by one or more OH, CO₂H, CO₂alk radicals, by an radical , a' and b' representing a hydrogen atom, an alkyl radical containing up to 8 carbon atoms, a' and b' being able to form a heterocycle optionally containing one or more additional heteroatoms and/or by a heterocycle containing one or more heteroatoms
or R2 represents a 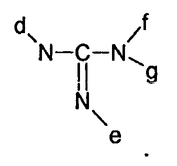 radical in which d, e, f and g represent a hydrogen atom or an alkyl radical containing up to 8 carbon atoms, in addition f and g being able to represent an acyl radical containing up to 8 carbon atoms, e and f also being able to form a cycle optionally containing one or more heteroatoms,
R3 represents a hydrogen atom, a methyl or hydroxyl radica,l
R4 represents a hydrogen atom or a hydroxyl radical,
R represents a linear or branched or cyclic chain containing up to 30 carbon atoms, optionally containing one or more heteroatoms, one or more heterocycles, or a linear, branched or cyclic acyl radical containing up to 30 carbon atoms optionally containing one or more heteroatoms and/or one or more heterocycles,
T represents a hydrogen atom, a methyl radical, a -CH₂CONH₂ radical, a - CH₂C≡N radical, a -(CH₂)₂NH₂ or a -(CH₂)₂Nalk₂⁺X⁻ radical, X being a halogen atom and alk an alkyl radical containing up to 8 carbon atoms,
Y represents a hydrogen atom, a hydroxyl radical or a halogen atom, or an - OSO₃H radical or one of the salts of this radical,
W represents a hydrogen atom or an OH radical,
Z represents a hydrogen atom or a methyl radical,
as well as the addition salts with acids of the products of formula (I).

2. The compounds of formula (I) defined in claim 1 in which T represents a hydrogen atom.

3. The compounds of formula (I) defined in claim 1 or 2 in which W represents a hydrogen atom.

4. The compounds of formula (I) defined in any one of claims 1 to 3, in which Z represents a methyl radical.

5. The compounds of formula (I) defined in any one of claims 1 to 4 in which Y represents a hydrogen atom.

6. The compounds of formula (I) defined in any one of claims 1 to 5 in which R3 represents a methyl radical.

7. The compounds of formula (I) defined in any one of claims 1 to 6 in which R4 represents a hydroxyl radical.

8. The compounds of formula (I) defined in any one of claims 1 to 7 in which R represents a radical.

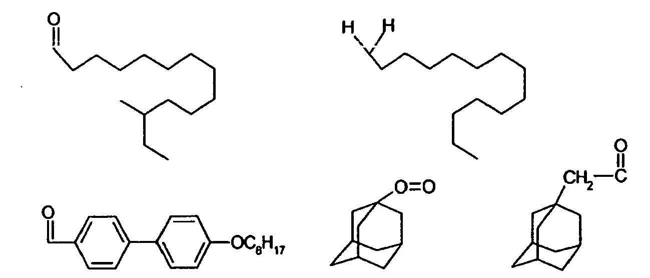

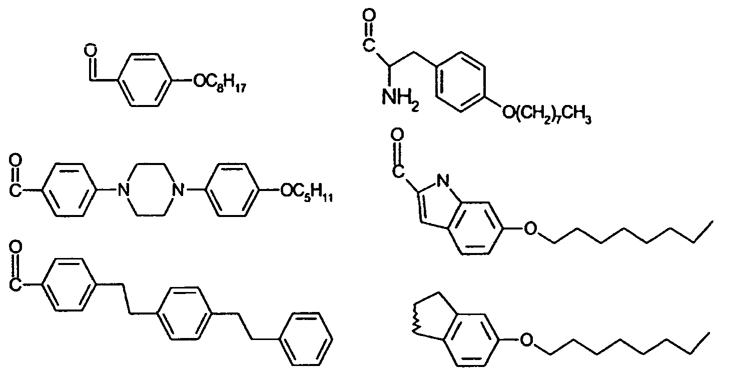

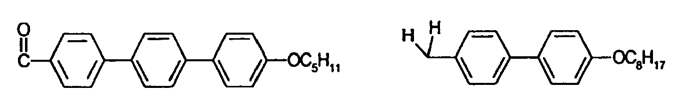

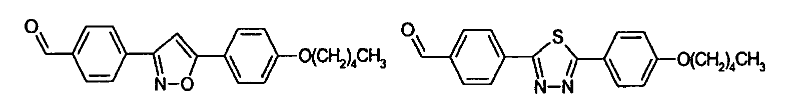

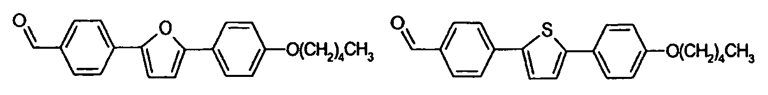

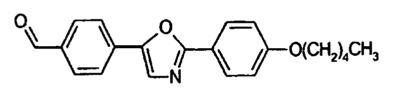

9. The compounds of formula (I) defined in claim 8, in which R represents a chain

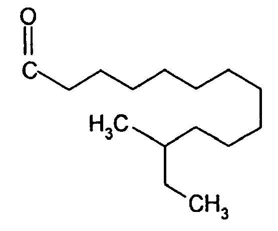

10. The compounds of formula (I) defined in claim 8 in which R represents a chain

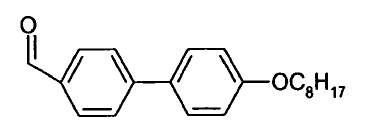

11. The compounds of formula (I) defined in any one of claims 1 to 10, in which R1 forms with the endocyclic carbon atom carrying the -NR1R2 radical, a double bond.

12. The compounds of formula (I) defined in claim 11, in which R2 represents the radical
-O(CH₂)ₙNY'₂
in which n represents an integer comprised between 1 and 8 and Y' represents a hydrogen atom or an alkyl radical containing up to 8 carbon atoms.

13. The compounds of formula (I) defined in claim 12, in which n represents the number 2.

14. The compounds of formula (I) defined in claim 8, in which R2 represents a radical

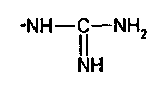

15. The compounds of formula (I) defined in any one of claims 1 to 10, in which R2 represents a radical
-(CH₂)ₚ NY"
in which Y" represents a hydrogen atom or an alkyl radical containing up to 8 carbon atoms and p represents an integer from 1 to 8.

16. The compounds of formula (I) defined in any one of claims 1 to 10 and 15, in which R 1 represents a hydrogen atom.

17. The compounds of formula (I) defined in claim 15, in which p represents the number 2.

18. The compounds of formula (I) defined in claim 1, the names of which follow:
. 1-[(S)-N2-(12-methyl-1-oxotetradecyl) 4-[[(3-piperidinyl) oxy] imino]-L-omithine] 4-[4-(4-hydroxyphenyl)-L-threonine]-5-L-serine echinocandine B,
. 1-[4-[(2-aminoethyl) amino]-N2-(12-methyl-1-oxotetra-decyl)-L-ornithine] 4-[4-(4-hydroxyphenyl)-L-threonine]-5-L-serine echinocandine B (isomer A and isomer B),
. 1-[4-[(aminoiminomethyl) hydrazono]-N2-(12-methyl-1-oxotetradecyl)-L-omithine] 4-[4-(4-hydroxyphenyl)-L-threonine]-5-L-serine echinocandine B,
. 1-[4-[(2-aminoethoxy) imino]-N2-(12-methyl-1-oxotetradecyl)-L-omithine] 4-[4-(4-hydroxyphenyl)-L-threonine]-5-L-serine echinocandine B and the corresponding isomer Z,
. 1-[4-[(2-aminoethyl)amino]-N2-[[4'-(octyloxy)[1,1'-biphenyl]-4-yl]carbonyl]-L-ornithine]-4-[4-(4-hydroxyphenyl)-L-threonine]-5-L-serine-echinocandine B (isomer A)
as well as their addition salts with acids.

19. A compound of formula (I) as defined in claim 1, in which T, W, Y and R₁ represent a hydrogen atom, Z and R₃ represent a methyl radical, R2 represents a - CH2-CH2-NH2 radical, R4 represents a hydroxyl radical, R represents a group 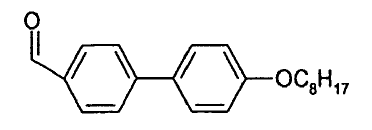 as well as their addition salts with acids.

20. Process for preparation of the compounds of formula (I) defined in any one of claims 1 to 18, **characterized in that** a compound of formula (II) 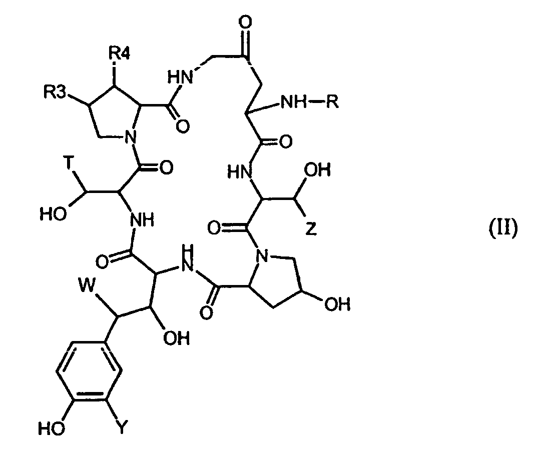 in which R, R3, R4, T, W, Y and Z retain their previous meaning, is subjected to the action of an amine or of an amine derivative capable of introducing
the 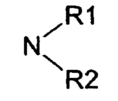 radical in which R1 and R2 retain their previous meaning
and, if desired, to the action of a reducing agent
and/or of a functionalization agent of the amine,
and/or of an acid in order to form the salt of the product obtained,
and/or of an agent able to separate the different isomers obtained,
and thus the expected compound of formula (I) is obtained 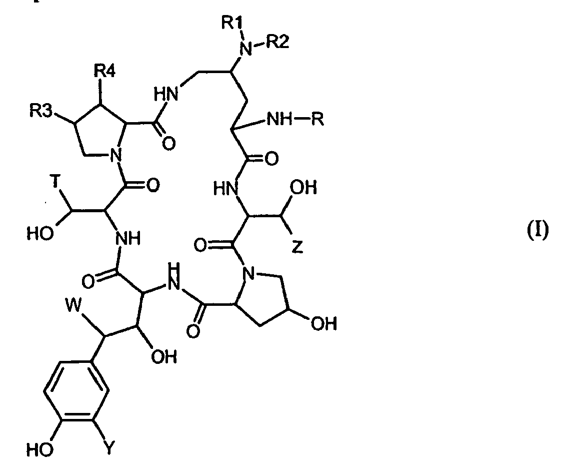 in which R1, R2, R3, R4, T, Y, W, R and Z retain their previous meaning and, if desired,the compound of formula (I) is subjected to the action of an acid to form the salt and, if desired, the different isomers thus obtained are separated.

21. As new chemical products, the compounds of formula (II) defined in claim 19.

22. As a new chemical product defined in claim 20, the compounds of formula (II) the name of which follows:
. 1([4-oxo-N2-(12-methyl-1-oxotetradecyl) L-ornithine] 4-[4-(4-hydroxyphenyl)-L-threonine]-5-L-serine-echinocandine B,
. 1-[N2-[[4'-(octyloxy)-[1,1'-biphenyl]-4-yl]carbonyl]-4-oxo-L-ornithine]-4-[4-(4-hydroxyphényl)-L-threonine]-5-L-serine-echinocandine B,
. 1-[N2-[[4-[4-[4-(pentyloxy) phenyl]-1-piperazinyl]-phenyl]-carbonyl]4-oxo-L-ornithine]-4-[4-(4-hydroxyphenyl)-L-threonine]-5-L-sérine echinocandine B.

23. Process according to claim 19 **characterized in that** a compound of formula (III) 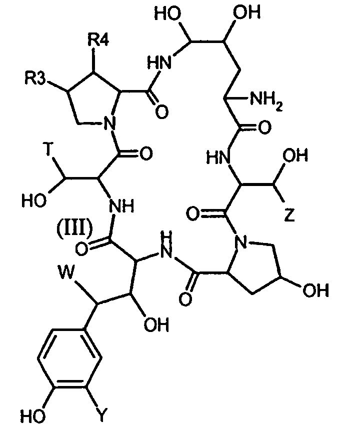 in which the different substituents retain their previous meaning is subjected to the action of an agent capable of replacing -NH₂ by -NHR, R retaining its previous meaning, in order to obtain the compound of formula (IV) 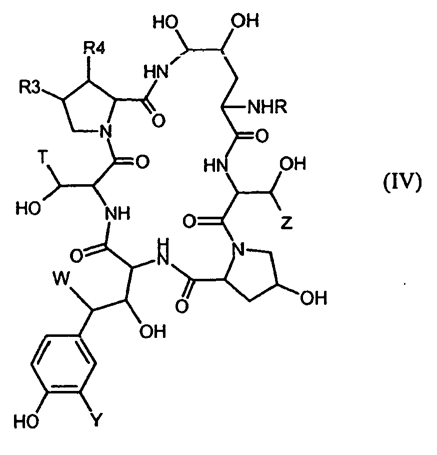 which is subjected to the action of trimethylsilyl iodide in order to obtain the corresponding compound of formula (II)

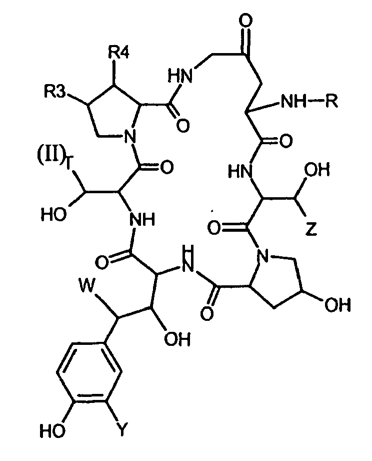

24. As anti-fungal compounds, the compounds of formula (I) defined in any one of claims 1 to 19, as well as their addition salts with acids.

25. The pharmaceutical compositions containing as a medicament, at least one compound of formula (I) defined in any one of claims 1 to 19, as well as their addition salts with pharmaceutically acceptable acids.
